(19)
Europäisches
Patentamt
European
Patent Office
Office européen
des brevets

(11) **EP 3 881 831 A1**

(12) # EUROPEAN PATENT APPLICATION

(43) Date of publication:
**22.09.2021 Bulletin 2021/38**

(51) Int Cl.:
**A61K 9/16** (2006.01)

(21) Application number: **21162145.3**

(22) Date of filing: **11.03.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.03.2020 JP 2020045860**

(71) Applicant: **Ricoh Company, Ltd.
Tokyo 143-8555 (JP)**

(72) Inventors:
• **MORINAGA, Tadahiko
Tokyo, 143-8555 (JP)**
• **MORITANI, Tatsuru
Tokyo, 143-8555 (JP)**

(74) Representative: **Fairbairn, Angus Chisholm
Marks & Clerk LLP
15 Fetter Lane
London EC4A 1BW (GB)**

(54) **SUSTAINED-RELEASE PARTICLES AND PRODUCTION METHOD THEREOF**

(57) Provided are sustained-release particles, each including a base material, and a physiologically active substance, wherein the ratio of the surface area of the sustained-release particles to the volume of the sustained-release particles is 0.6 or less.

EP 3 881 831 A1

**Description**

BACKGROUND OF THE INVENTION

Field of the Invention

The present disclosure is related to sustained-release particles and a production method thereof.

Description of the Related Art

Sustained-release pharmaceutical preparations, which can gradually release a physiologically active substance, such as a drug, in vivo and be allowed to be absorbed, have been developed.

It is important for a sustained-release pharmaceutical preparation to prevent initial burst and control to release the physiologically active substance at a constant speed after administration. The initial burst is a phenomenon that a large amount of the physiologically active substance is dissolved within a few hours after administration inside a body. When an amount of the physiologically active substance dissolved is large due to the initial burst, a body may be adversely affected by side effects of the physiologically active substance, or it may be difficult to control an appropriate administration cycle of the physiologically active substance. Therefore, there have been various attempts to prevent initial burst.

For example, proposed are microspheres, in which a type and amount of a physiologically active substance for use, a type, amount and properties of a base material for use, and a weight average particle diameter of the microspheres are controlled (see, for example, Japanese Patent No. 5423003).

SUMMARY OF THE INVENTION

According to one aspect of the present disclosure, sustained-release particles each includes a physiologically active substance and a base material. The ratio of the surface area of the sustained-release particles to the volume of the sustained-release particles is 0.6 or less.

BRIEF DESCRIPTION OF THE DRAWINGS

FIG. 1 is a schematic cross-sectional view illustrating one example of a liquid column resonance droplet-ejecting unit;

FIG. 2 is a schematic view illustrating one example of an apparatus for producing particles;

FIG. 3 is a schematic view illustrating another example of the apparatus for producing particles;

FIG. 4A is a schematic view illustrating one example of an apparatus for producing particles, where the apparatus can impart a flow of a poor solvent to an ejection area of the droplet-ejecting unit;

FIG. 4B is an enlarged view of the section including the droplet-ejecting unit (section marked with a dashed line) of FIG. 4A;

FIG. 5 is a schematic view illustrating an apparatus for producing particles including a good solvent removing unit configured to remove a good solvent;

FIG. 6 is a graph depicting one example of a particle size distribution of the particles produced by the method of the second embodiment, and a particle size distribution of the particles produced by spray drying;

FIG. 7 is a schematic view illustrating one example of an apparatus for producing particles;

FIG. 8 is a schematic cross-sectional view illustrating one example of a droplet-ejecting unit used in the apparatus for producing particles; and

FIG. 9 is a schematic cross-sectional view illustrating another example of the droplet-ejecting unit used in the apparatus for producing particles.

DESCRIPTION OF THE EMBODIMENTS

(Sustained-release particles)

The sustained-release particles of the present disclosure each include a base material and a physiologically active substance. The ratio of the surface area of the sustained-release particles to the volume of the sustained-release particles is 0.6 or less. The sustained-release particles may further include other materials according to the necessity.

The present disclosure has an object to provide sustained-release particles, which can prevent initial burst and have excellent sustained releasing performance suitable for medicine etc.

The present disclosure can provide sustained-release particles, which can prevent initial burst and have excellent sustained releasing performance suitable for medicine etc.

The present inventors have studied on sustained-release particles. As a result, the present inventors have found the following insights.

In the art, characteristics of shapes of particles are judged based on indexes, such as a volume average particle diameter and a weight average particle diameter. However, an amount of fine particles (particles having the volume average particle diameter of 5.0 μm or less), which are a main factor for causing initial burst, cannot be easily represented by such indexes, and therefore it has been a problem that it may not be able to understand how much fine particles are included in obtained (bulk) particles.

The present inventors have found that the surface area of particles is related to initial burst of the particles as well as a size of the particles.

Since the ratio (surface area/volume) of the sustained-release particles of the present disclosure is 0.6 or less, the sustained-release particles do not include fine particles, and can realize sustained-release with suppressing initial burst.

In the present specification, the term "particle(s)" may be also referred to as "microcapsule(s)" or "microparticle(s)."

The particles of the present disclosure each include at least one base material, and a physiologically active substance having bioactivity, and may further include other materials according to the necessity. The physiologically active substance is not particularly limited as long as the physiologically active substance exhibits some bioactivity in vivo. As a preferred embodiment, the physiologically active substance has a characteristic that bioactivity thereof changes by chemical or physical stimuli, such as heating, cooling, shaking, and a change in pH.

In the present specification, the term "particles" means a group of a granular composition including the base material and the physiologically active substance, unless otherwise stated. The particles of the present disclosure are sustained-release particles that continuously release a drug over a long-period of time.

In the present specification, the term "base material" means a component included in the particles, and is a material that is a base constituting each particle.

In the present specification, the term "physiologically active substance" means an active ingredient used for exhibiting physiological effects on living matter. Examples of the physiologically active substance include: low molecular weight compounds, such as pharmaceutical compounds, edible compounds, and cosmetic compounds; and macromolecular compounds including biopolymers, such as proteins (e.g., antibodies and enzymes), and nucleic acids (e.g., DNA and RNA). Moreover, the term "physiological effect" means an effect obtained as a result of bioactivity of the physiologically active substance exhibited at a target site, and examples thereof include quantitative and/or qualitative changes or influences on organisms, tissues, cells, proteins, DNAs, RNAs, etc. Moreover, the term "bioactivity" means that the physiologically active substance functions to change or affect a target site (e.g., target tissues). For example, the target site is preferably a receptor etc. present on a surface of a cell or within a cell. In this case, a signal is transmitted to a cell by bioactivity, which is binding of the physiologically active substance to a specific receptor, and as a result, a physiological effect is exhibited.

The physiologically active substance may be a substance which is turned into a mature form by an enzyme in vivo and is bonded to a specific receptor as the mature form to thereby exhibit a physiological effect. In this case, a substance before turned into a mature form is also included in the scope of the physiologically active substance.

The physiologically active substance may be a substance created by a living organism (human or creatures other than human), or an artificially synthesized substance.

Examples of characteristic physical properties of the particles of the present disclosure include the ratio (surface area/volume) of a surface area to a volume, a particle size distribution, and particle diameters.

-Surface area/volume-

The ratio (surface area/volume) of the sustained-release particles of the present disclosure is 0.6 or less, preferably 0.5 or less, and more preferably 0.3 or less. When the ratio (surface area/volume) is 0.6 or less, the sustained-release particles do not include dine particles, and initial burst can be suppressed.

Examples of a calculation method of the ratio (surface are/volume) include a measurement method using a fiber-optics particle analyzer (FPAR-1000, available from Otsuka Electronic Co., Ltd.) according to a dynamic light scattering method, and a method where the ratio is calculated according to the formula below using a surface area and a volume determined from a number average particle diameter Dn obtained by means of a laser scattering particle size distribution analyzer (LA-960, available from HORIBA, Ltd.).

The ratio (surface area/volume) is calculated by the following equation.

Surface area/volume = (sum of surface areas of particles each in the size of the number average particle diameter Dn)/(sum of volumes of particles each in the size of the number average particle diameter Dn)

The sum of surface areas of particles each in the size of the number average particle diameter Dn is calculated by the following equation.

Sum of surface areas of particles each in the size of the number average particle diameter Dn = sum of (surface areas of equivalent spheres each having a diameter identical to the number average particle diameter Dn of particles × abundance ratio)

The sum of volumes of particles each in the size of the number average particle diameter Dn is calculated by the following equation.

Sum of volumes of particles each in the size of the number average particle diameter Dn = sum of (volumes of equivalent spheres each having a diameter identical to the number average particle diameter Dn of particles × abundance ratio)

As a measurement sample for the above-mentioned device, used is a dispersion liquid prepared by adding 0.1 g of DRIWEL K (DRIWEL, available from FUJIFILM Corporation) and 5.0 g of water to 0.003 g of the sample particles, and dispersing the mixture for 3 minutes by ultrasonic waves.

-Measurement conditions-

Transmittance (R): 85% to 95%
Transmittance (B): 70% to 90%
Algorithm option: standard mode

-Particle size distribution-

The particles of the present disclosure preferably have a narrow particle size distribution. Specific examples of an index for representing a breadth of a particle size distribution include Relative Span Factor (R.S.F) and a ratio (Dv/Dn) of a volume average particle diameter (Dv) to a number average particle diameter (Dn). For example, R.S.F. is preferably in the range of 0 < (R.S.F) ≤ 1.2, and the ratio Dv/Dn is preferably 1.00 or greater but 1.50 or less. When the particle size distribution is within the above-mentioned ranges, a proportion of coarse particles relative to a targeted particle size is small. Therefore, filtration sterilization by filtration can be simply and effectively performed without clogging a sterilization filter with the particles, when the particles are added to a pharmaceutical composition and need to be sterilized by filtration before use. Since the size of the particles is homogeneous, moreover, an amount of the physiologically active substance and an amount of the base material of each particle, and a surface area of each particle is consistent. As a result, a substantially equal amount of the physiologically active substance is dissolved from each of the particles, and therefore the particles, with which sustained release of the physiologically active substance can be highly controlled, can be obtained. Since the size of the particles is homogeneous, furthermore, formation of fine particles formed only of the physiologically active substance, without including the base material, can be suppressed, and therefore sustained-release particles that can prevent initial burst can be obtained.

--Relative Span Factor (R.S.F)--

In the present disclosure, "Relative Span Factor (R.S.F)" is defined as (D90-D10)/D50. The D90 denotes a cumulative 90% by number from a small particle side of a cumulative particle size distribution, the D50 denotes a cumulative 50% by number from the small particle side of the cumulative particle size distribution, and the D10 denotes a cumulative 10% by number from the small particle side of the cumulative particle size distribution. (R.S.F) is preferably 0 < (R.S.F) ≤ 1.2, more preferably 0 < (R.S.F) ≤ 1.0, and even more preferably 0 < (R.S.F) ≤ 0.6.

Examples of a measurement method of (R.S.F) include a measurement method using a fiber-optics particle analyzer (FPAR-1000, available from Otsuka Electronic Co., Ltd.) according to a dynamic light scattering method, and a measurement method using a laser scattering particle size distribution analyzer (LA-960, available from HORIBA, Ltd.).

--Volume average particle diameter (Dv)/number average particle diameter (Dn)--

The ratio, volume average particle diameter (Dv)/number average particle diameter (Dn), is a value obtained by dividing the volume average particle diameter (Dv) by the number average particle diameter (Dn). The ratio (Dv)/(Dn) is preferably 1.00 or greater but 1.50 or less, and more preferably 1.00 or greater but 1.20 or less.

Examples of a measurement method of the volume average particle diameter (Dv), and the number average particle diameter (Dn) include measurement methods using a laser diffraction/scattering particle size distribution analyzer (device name: MICROTRAC MT3000II, available from MicrotracBEL Corp.) or a laser scattering particle size distribution analyzer (LA-960, available from HORIBA, Ltd.).

-Particle diameter-

The number average particle diameter (Dn) of the particles is preferably 1 $\mu$m or greater but 50 $\mu$m or less, and more preferably 10 $\mu$m or greater but 30 $\mu$m or less, but the number average particle diameter (Dn) thereof may be appropriately selected depending on the intended purpose.

When the number average particle diameter (Dn) is 1 $\mu$m or greater but 50 $\mu$m or less, a sufficient amount of the physiologically active substance is encapsulated in each particle. For example, therefore, particles that can gradually release the physiologically active substance over a long period of time can be produced. Note that, the volume average particle diameter (Dv) is more preferably 1 $\mu$m or greater but 50 $\mu$m or less.

The measurement method of the number average particle diameter (Dn) of the particles include a measurement method using a fiber-optics particle analyzer (FPAR-1000, available from Otsuka Electronic Co., Ltd.) according to a dynamic light scattering method, and a measurement method using a laser diffraction / scattering particle size distribution analyzer (device name: MICROTRAC MT3000II, available from MicrotracBEL Corp.) or laser scattering particle size distribution analyzer (LA-960, available from HORIBA, Ltd.).

Next, embodiments of particles will be described. Generally, embodiments of the particles each including the base material and the physiologically active substance include capsule particles, which are an embodiment where the physiologically active substance is encapsulated in the base material, carrier particles, in which the physiologically active substance is carried on a surface of each particle of the base material, and particles of other embodiments.

Examples of the capsule particles include dispersion capsule particles, in each of which the physiologically active substance is included in the state where the physiologically active substance is dispersed in the base material, and maldistribution capsule particles, where the physiologically active substance is unevenly distributed and included in the base material. The term "included" associated with the capsule particles is not particularly limited, as long as it means that the physiologically active substance is temporarily or continuously retained in the base material.

The dispersion capsule particles are not particularly limited as long as the physiologically active substance is dispersed and included in the base material. The physiologically active substance may not be uniformly dispersed in the base material.

The maldistribution capsule particles are particles, in each of which the physiologically active substance is unevenly distributed and included in the base material. In other words, it is an embodiment where the physiologically active substance is included in the base material by arranging the physiologically active substance to be substantially separated from the base material within each particle. Examples of an embodiment of the maldistribution capsule particles include an embodiment of a particle where the physiologically active substance constitutes a core, and the base material constitutes a shell covering the core. Examples of the maldistribution capsule particles include liposomes, micelles, and coated particles.

When each particle includes a plurality of base materials, and one of the base materials is unevenly arranged within each particle, the degree of dispersion of the physiologically active substance may be different depending on the type of the base material in which the physiologically active substance is included. Examples of the dispersion capsule particles include particles of the present disclosure, particles produced by emulsion solvent diffusion (ESD), and particles produced by spray drying.

Moreover, the particles of the present disclosure may include two or more base materials. When the particles include two or more base materials, one base material may be included and locally arranged at the surface side of each particle. In this case, the physiologically active substance is included and dispersed in both the base material included and locally arranged at the surface side of each particle (may be referred to as a "surface base material" hereinafter), and the base material(s) other than the surface base material (may be referred to as an "inner base material"), even though there may be a difference in the degree of dispersion.

Specific examples of such embodiment include: an embodiment where the physiologically active substance is included more in the surface base material; and an embodiment where the physiologically active substance is included more in the inner base material. Among the above-listed example, the embodiment where the physiologically active substance is included more in the inner base material is preferable. Since the physiologically active substance is included more in the inner base material, the sustained-release particles, where the dissolution speed of the physiologically active substance is controlled, can be produced.

A method for confirming the embodiment where the particles include two or more base materials, and one of the two or more base materials is included and locally arranged at the surface side of each particle is not particularly limited and may be appropriately selected depending on the intended purpose.

One example of the confirmation method is a method where cross-sections of the particles are observed by a scanning electron microscope, a transmission electron microscope, or a scanning probe microscope.

Moreover, another example of the confirmation method is a method where components of the surface base material are measured by time-of-flight secondary ion mass spectrometry, and the particles are confirmed if the measured components can be judged as being different from the components of the inner base material.

As another confirmation method, moreover, a pre-treatment, such as electron staining and a dissolution treatment, can be performed. In the case where the particles each include a base material of a water-soluble component, and a base material of a water-insoluble component, for example, the cross-sections of the particles are immersed in water, and the cross-sections of the particles from which the water-soluble component is completely dissolved is observed by a scanning electron microscope to judge the remaining part of the cross-section of each particle as the water-insoluble component, and the void as the water-soluble component, to thereby confirm the particles.

<Base material>

The base material is a material that is a base for constituting particles. Therefore, the base material is preferably a solid at room temperature.

The base material may be a low-molecular weight material or a high-molecular weight material, as long as the base material does not adversely affect the physiologically active substance included in the particles together with the base material. The particles of the present disclosure are preferably particles applied for living matter. Therefore, the base material is preferably a material that is not toxic to living matter.

The low-molecular weight material is preferably a compound having a weight average molecular weight of less than 15,000.

The high-molecular weight material is preferably a compound having a weight average molecular weight of 15,000 or greater.

As described above, the base material for use may be one base material, or two or more base materials. Any of the base materials described below may be used in combination. The base material for use in the present disclosure preferably include at least one polylactic acid, or a polylactic acid-glycolic acid copolymer.

-Low-molecular weight material-

The low-molecular weight material is not particularly limited and may be appropriately selected depending on the intended purpose. Examples of the low-molecular weight material include lipids, saccharides, cyclodextrins, amino acids, and organic acids. The above-listed examples may be used alone or in combination.

-- Lipids--

The lipids are not particularly limited and may be appropriately selected depending on the intended purpose. Examples of the lipids include medium or long chain monoglyceride, medium or long chain diglyceride, medium or long chain triglyceride, phospholipid, vegetable oil (e.g., soybean oil, avocado oil, squalene oil, sesame oil, olive oil, corn oil, rapeseed oil, safflower oil, and sunflower oil), fish oil, seasoning oil, water-insoluble vitamins, fatty acids, mixtures thereof, and derivatives thereof. The above-listed examples may be used alone or in combination.

--Saccharides--

The saccharides are not particularly limited and may be appropriately selected depending on the intended purpose. Examples of the saccharides include: monosaccharides and polysaccharides, such as glucose, mannose, idose, galactose, fucose, ribose, xylose, lactose, sucrose, maltose, trehalose, turanose, raffinose, maltotriose, acarbose, cyclodextrins, amylose (starch), and cellulose; sugar alcohols (polyols), such as glycerin, sorbitol, lactitol, maltitol, mannitol, xylitol, and erythritol; and derivatives thereof.. The above-listed examples may be used alone or in combination.

--Cyclodextrins--

The cyclodextrins are not particularly limited and may be appropriately selected depending on the intended purpose. Examples of the cyclodextrins include hydroxypropyl-β-cyclodextrin, β-cyclodextrin, γ-cyclodextrin, α-cyclodextrin, and cyclodextrin derivatives. The above-listed examples may be used alone or in combination.

--Amino acids--

The amino acids are not particularly limited and may be appropriately selected depending on the intended purpose. Examples of the amino acids include valine, lysine, leucine, threonine, isoleucine, asparagine, glutamine, phenylalanine, aspartic acid, serine, glutamic acid, methionine, arginine, glycine, alanine, tyrosine, proline, histidine, cysteine, tryptophan, and derivatives thereof. The above-listed examples may be used alone or in combination.

--Organic acids--

The organic acids are not particularly limited and may be appropriately selected depending on the intended purpose. Examples of the organic acids include adipic acid, ascorbic acid, citric acid, fumaric acid, gallic acid, glutaric acid, lactic acid, malic acid, maleic acid, succinic acid, tartaric acid, and derivatives thereof. The above-listed examples may be used alone or in combination.

-High-molecular weight material-

The high-molecular weight material is not particularly limited and may be appropriately selected depending on the intended purpose. Examples of the high-molecular weight material include water-soluble cellulose, polyalkylene glycol, poly(meth)acrylamide, poly(meth)acrylic acid, poly(meth)acrylic acid ester, polyallylamine, polyvinyl pyrrolidone, polyvinyl alcohol, polyvinyl acetate, biodegradable resins, polyglycolic acid, polyamino acid, gelatin, protein (e.g., fibrin), polysaccharides, and derivatives thereof. The above-listed examples may be used alone or in combination.

--Water-soluble cellulose--

The water-soluble cellulose is not particularly limited and may be appropriately selected depending on the intended purpose. Examples of the water-soluble cellulose include: alkyl cellulose, such as methyl cellulose, and ethyl cellulose; hydroxyalkyl cellulose, such as hydroxyethyl cellulose and hydroxypropyl cellulose; and hydroxyalkyl alkyl cellulose, such as hydroxyethyl methyl cellulose and hydroxypropyl methyl cellulose. The above-listed examples may be used alone or in combination. Among the above-listed examples, hydroxypropyl cellulose and hydroxypropyl methyl cellulose are preferable, and hydroxypropyl cellulose is more preferable because of high biocompatibility and high solubility to a solvent for producing particles.

---Hydroxypropyl cellulose---

As the hydroxypropyl cellulose, various products having different viscosities are available on the market from manufacturers. Any of such commercial products can be used for the base material of the present disclosure. A viscosity of a 2% by mass hydroxypropyl cellulose aqueous solution (20°C) is not particularly limited and may be appropriately selected depending on the intended purpose. The viscosity thereof is preferably 2.0 mPa•s (centipoise, cps) or greater but 4,000 mPa•s (centipoise, cps) or less.
Moreover, it seems that the viscosity of the hydroxypropyl cellulose depends on a weight average molecular weight, substitution degree, and molecular weight of the hydroxypropyl cellulose.
The weight average molecular weight of the hydroxypropyl cellulose is not particularly limited and may be appropriately selected depending on the intended purpose. The weight average molecular weight thereof is preferably 15,000 or greater but 400,000 or less. The weight average molecular weight thereof can be measured, for example, by gel permeation chromatography (GPC).
A commercial product of the hydroxypropyl cellulose is not particularly limited and may be appropriately selected depending on the intended purpose. Examples of the commercial product thereof include HPC-SSL having a molecular weight of 15,000 or greater but 30,000 or less and viscosity of 2.0 mPa•s or greater but 2.9 mPa•s or less, HPC-SL having a molecular weight of 30,000 or greater but 50,000 or less and viscosity of 3.0 mPa•s or greater but 5.9 mPa•s or less, HPC-L having a molecular weight of 55,000 or greater but 70,000 or less and viscosity of 6.0 mPa•s or greater but 10.0 mPa•s or less, HPC-M having a molecular weight of 110,000 or greater but 150,000 or less and viscosity of 150 mPa•s or greater but 400 mPa•s or less, and HPC-H having a molecular weight of 250,000 or greater but 400,000 or less and viscosity of 1,000 mPa•s or greater but 4,000 mPa•s or less (all available from Nippon Soda Co., Ltd.). The above-listed examples may be used alone or in combination. Among the above-listed examples, HPC-SSL having a molecular weight of 15,000 or greater but 30,000 or less and viscosity of 2.0 mPa•s or greater but 2.9 mPa•s or less is preferable. The molecular weights of the above-listed commercial products can be measured by gel permeation chromatography (GPC), and the viscosity thereof is measured using a 2% by mass aqueous solution (20°C).

An amount of the hydroxypropyl cellulose is not particularly limited and may be appropriately selected depending on the intended purpose. The amount of the hydroxypropyl cellulose is preferably 50% by mass or greater, more preferably 50% by mass or greater but 99% by mass or less, even more preferably 75% by mass or greater but 99% by mass or less, and particularly preferably 80% by mass or greater but 99% by mass or less, relative to a mass of the base material.

--Polyalkylene glycol--

The polyalkylene glycol is not particularly limited and may be appropriately selected depending on the intended purpose. Examples thereof include polyethylene glycol (PEG), polypropylene glycol, polybutylene glycol, and copolymers thereof. The above-listed examples may be used alone or in combination.

--Poly(meth)acrylamide--

The poly(meth)acrylamide is not particularly limited and may be appropriately selected depending on the intended purpose. Examples thereof include polymers of monomers, such as N-methyl(meth)acrylamide, N-ethyl(meth)acrylamide, N-propyl(meth)acrylamide, N-buthyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl)(meth)acrylamide, N-(sulfamoylphenyl)(meth)acrylamide, N-(phenylsulfonyl)(meth)acrylamide, N-(tolylsulfonyl)(meth)acrylamide, N,N-dimethyl(meth)acrylamide, N-methyl-N-phenyl(meth)acrylamide, and N-hydroxyethyl-N-methyl(meth)acrylamide. Any of the above-listed monomers may be polymerized alone, or a combination thereof may be polymerized together. Moreover, the above-listed polymers may be used alone or in combination.

--Poly(meth)acrylic acid--

The poly(meth)acrylic acid is not particularly limited and may be appropriately selected depending on the intended purpose. Examples thereof include: homopolymers, such as polyacrylic acid and polymethacrylic acid; and copolymers, such as acrylic acid-methacrylic acid copolymer. The above-listed examples may be used alone or in combination.

--Poly(meth)acrylic acid ester--

The poly(meth)acrylic acid ester is not particularly limited and may be appropriately selected depending on the intended purpose. Examples thereof include polymers of monomers, such as ethylene glycol di(meth)acrylate, diethylene glycol di(meth)acrylate, propylene glycol di(meth)acrylate, glycerol poly(meth)acrylate, polyethylene glycol (meth)acrylate, trimethylolpropane tri(meth)acrylate, pentaerythritol tetra(meth)acrylate, and 1,3-butyleneglycol di(meth)acrylate. Any of the above-listed monomers may be polymerized alone, or a combination thereof may be polymerized together. Moreover, the above-listed polymers may be used alone or in combination.

--Polyallylamine--

The polyallylamine is not particularly limited and may be appropriately selected depending on the intended purpose. Examples thereof include diallylamine, and triallylamine. The above-listed examples may be used alone or in combination.

--Polyvinyl pyrrolidone--

As the polyvinyl pyrrolidone, a commercial product may be used. The commercial product of the polyvinyl pyrrolidone is not particularly limited and may be appropriately selected depending on the intended purpose. Examples of the commercial product thereof include PLASDONE C-15 (available from ISP TECHNOLOGIES); KOLLIDON VA 64, KOLLIDON K-30, and KOLLIDON CL-M (all available from KAWARLAL); and KOLLICOAT IR (available from BASF). The above-listed examples may be used alone or in combination.

--Polyvinyl alcohol--

The polyvinyl alcohol is not particularly limited and may be appropriately selected depending on the intended purpose. Examples of the polyvinyl alcohol include silanol-modified polyvinyl alcohol, carboxyl-modified polyvinyl alcohol, and acetoacetyl-modified polyvinyl alcohol. The above-listed examples may be used alone or in combination.

--Polyvinyl acetate--

The polyvinyl acetate is not particularly limited and may be appropriately selected depending on the intended purpose. Examples thereof include vinyl acetate-crotonic acid copolymer, and vinyl acetate-itaconic copolymer. The above-listed examples may be used alone or in combination.

--Biodegradable resin--

The biodegradable resin is not particularly limited and may be appropriately selected depending on the intended purpose. Examples of the biodegradable resin include biodegradable polyester. Examples of the biodegradable polyester include: polylactic acid; poly-e-caprolactone; succinate-based polymers (polylactic acid-glycolic acid copolymers), such as polyethylene succinate, polybutylene succinate, and polybutylene succinate adipate; polyhydroxyalkanoate, such as polyhydroxypropionate, polyhydroxy butylate, and polyhydroxy valerate; and polyglycolic acid. The above-listed examples may be used alone or in combination. Among the above-listed examples, polylactic acid is preferable because the polylactic acid has high biocompatibility, and a physiologically active substance included therein can be gradually released.

---Polylactic acid---

The weight average molecular weight of the polylactic acid is not particularly limited and may be appropriately selected depending on the intended purpose. The weight average molecular weight thereof is preferably 5,000 or greater but 100,000 or less, more preferably 10,000 or greater but 70,000 or less, even more preferably 10,000 or greater but 50,000 or less, and particularly preferably 10,000 or greater but 30,000 or less.

An amount of the polylactic acid is not particularly limited and may be appropriately selected depending on the intended purpose. The amount of the polylactic acid is preferably 50% by mass or greater, more preferably 50% by mass or greater but 99% by mass or less, even more preferably 75% by mass or greater but 99% by mass or less, and particularly preferably 80% by mass or greater but 99% by mass or less, relative to a mass of the base material.

---Polyglycolic acid---

The polyglycolic acid is not particularly limited and may be appropriately selected depending on the intended purpose. Examples thereof include: a lactic acid-glycolic acid polymer that is a copolymer including a structural unit derived from lactic acid and a structural unit derived from glycolic acid; a glycolic acid-caprolactone copolymer that is a copolymer including a structural unit derived from glycolic acid and a structural unit derived from caprolactone; and a glycolic acid-trimethylene carbonate copolymer that is a copolymer including a structural unit derived fromglycolic acid and a structural unit derived from trimethylene carbonate. The above-listed examples may be used alone or in combination. Among the above-listed examples, a lactic acid-glycolic acid polymer is preferable because the lactic acid-glycolic acid polymer has high, can gradually release a physiologically active substance contained therein, and can store the physiologically active substance contained therein over a long period of time.

As the lactic acid-glycolic acid copolymer, for example, any of PURASORB PDLG5010, PURASORB PDLG7510, PURASORB PDLG7507, PURASORB PDLG5002A, PURASORB PDLG5002, and PURASORB PDLG7502A (available from Corbion), or any of RG502, RG502H, RG503, RG503H, RG504, and RG504H (available from Sigma-Aldrich) can be used.

The weight average molecular weight of the lactic acid-glycolic acid copolymer is not particularly limited and may be appropriately selected depending on the intended purpose. The weight average molecular weight thereof is preferably from 2,000 through 250,000, more preferably from 2,000 through 100,000, even more preferably from 3,000 through 50,000, and particularly preferably from 5,000 through 10,000.

A molar ratio (L:G) of the structural unit (L) derived from lactic acid to the structural unit (G) derived from the glycolic acid in the lactic acid-glycolic acid copolymer is not particularly limited and may be appropriately selected depending on the intended purpose. The molar ratio (L:G) is preferably from 1:99 through 99:1, more preferably from 25:75 through 99:1, even more preferably from 30:70 through 90:10, and particularly preferably from 50:50 through 85:15.

An amount of the lactic acid-glycolic acid copolymer is not particularly limited and may be appropriately selected depending on the intended purpose. The amount of the lactic acid-glycolic acid copolymer is preferably 50% by mass or greater, more preferably 50% by mass or greater but 99% by mass or less, even more preferably 75% by mass or greater but 99% by mass or less, and particularly preferably 80% by mass or greater but 99% by mass or less, relative to a mass of the base material.

--Polyamino acid--

The polyamino acid is not particularly limited and may be appropriately selected depending on the intended purpose.

The polyamino acid may be a polymer of any appropriately selected combination of the above-listed amino acids, but preferably a polymer of single amino acid. Preferable examples of the polyamino acid include: homopolymers of amino acid, such as poly-$\alpha$-glutamic acid, poly-$\gamma$-glutamic acid, polyaspartic acid, polylysine, polyarginine, polyornithine, and polyserine; and copolymers thereof. The above-listed examples may be used alone or in combination.

--Gelatin--

The gelatin is not particularly limited and may be appropriately selected depending on the intended purpose. Examples of the gelatin include alkali processed gelatin, acid processed gelatin, gelatin hydrolysate, enzymatically dispersed gelatin, and derivatives thereof. The above-listed examples may be used alone or in combination.

A natural dispersant polymer used for the gelatin derivative is not particularly limited and may be appropriately selected depending on the intended purpose. Examples thereof include protein, polysaccharides, and nucleic acid. Examples thereof also include a copolymer including a natural dispersant polymer or a synthetic dispersant polymer. The above-listed examples may be used alone or in combination.

The gelatin derivative refers to gelatin derivatized by covalently binding a hydrophobic group to a gelatin molecule.

The hydrophobic group is not particularly limited and may be appropriately selected depending on the intended purpose. Examples of the hydrophobic group include: polyesters, such as polylactic acid, polyglycolic acid, and poly-$\varepsilon$-caprolactone; lipids, such as cholesterol and phosphatidyl ethanolamine; aromatic groups including alkyl groups and benzene rings; heterocyclic aromatic groups; and mixtures thereof.

--Protein--

The protein is not particularly limited and may be appropriately selected depending on the intended purpose, as long as the protein for use does not adversely affect the bioactivity of the physiologically active substance. Examples of the protein include collagen, fibrin, and albumin. The above-listed examples may be used alone or in combination.

--Polysaccharides--

The polysaccharides is not particularly limited and may be appropriately selected depending on the intended purpose. Examples of the polysaccharides include chitin, chitosan, hyaluronic acid, alginic acid, starches, and pectin. The above-listed examples may be used alone or in combination.

Since the base material is preferably a material with which resulting particles including the material can be added to a pharmaceutical preparation, a functional food product, and a functional cosmetic product, the base material is preferably a material that does not have ecotoxicity, particularly, a biodegradable material, such as a biodegradable polymer.

An amount of the base material is preferably 50% by mass or greater but 99.9% by mass or less, and more preferably 70% by mass or greater but 95% by mass or less, relative to a total amount of the particles. When the amount of the base material is 50% by mass or greater but 99.9% by mass or less relative to a total amount of the particles, a drug (physiologically active substance) can be encapsulated in the base material and thus the drug can be gradually released.

<Physiologically active substance>

The physiologically active substance is an active ingredient used for exhibiting a bioactive effect on living matter. Examples of the physiologically active substance include physiologically active substances included in pharmaceutical compositions, physiologically active substances included in functional food products, and physiologically active substances included in functional cosmetic products. The above-listed examples may be used alone or in combination.

-Physiologically active substance included in pharmaceutical composition-

The physiologically active substance included in a pharmaceutical composition is not particularly limited and may be appropriately selected depending on the intended purpose. Examples thereof include polypeptide (e.g., nucleic acid, and protein), saccharides, lipids, and a low-molecular weight compound. The above-listed examples may be used alone or in combination.

--Nucleic acid--

Examples of the nucleic acid typically include DNA, RNA, and a combination thereof, which may be substituted with chemically-modified nucleic acid part of entire sequence of which is chemically modified. Moreover, examples of the nucleic acid also include chemically synthetized nucleic acid analogues, such as peptide nucleic acid (PNA), and morpholino antisense oligo.

When the aim is to suppress expression of a target gene, for example, examples of the nucleic acid include an antisense nucleic acid complementary to a transcription product of a target gene or part of the transcription product thereof, a nucleic acid having ribozyme activity that causes specific cleavage of a transcription product of a target gene, a short-chain nucleic acid having a function of suppressing expression of a target gene due to RNAi interference, microRNA (miRNA), aptamer, locked nucleic acid obtained by modifying oligonucleotide.

--Polypeptide--

A polypeptide is a polymer formed of a plurality of amino acids. Among polypeptides, those having a higher-order structure and exhibiting a function derived from the higher-order structure are particularly referred to as proteins.

For example, the polypeptide includes both a polypeptide that is not being modified from the original state exiting in the nature, and a polypeptide that is modified

Examples of the modification include acetylation, acylation, ADP-ribosylation, amidation, a covalent bond of flavin, a covalent bond of a heme moiety, a covalent bond of nucleotide or a nucleotide derivative, a covalent bond of lipid or a lipid derivative, a covalent bond of phosphatidylinositol, crosslink, cyclization, formation of a disulfide bond, demethylation, formation of covalent crosslink, formation of cystine, formation of pyroglutamate, formylation, γ-carboxylation, glycosylation, formation of GPI anchor, hydroxylation, iodination, methylation, myristoylation, oxidation, a protein decomposition treatment, phosphorylation, prenylation, racemization, selenoylation, sulfation, tRNA mediation addition of amino acid to protein, such as arginylation, and ubiquitination.

When the aim is to inhibit or suppress a function of a target protein, examples of the protein include a target protein variant that is dominant-negative to the target protein, and antibody that binds to a target protein.

The antibody may be a polyclonal antibody or monoclonal antibody, or a polyspecific antibody, such as a bispecific antibody and a trispecific antibody, as long the antibody binds to a target protein. The antibody may be any antibody derived from animals, as long as the antibody exhibits a physiological effect. The antibody is preferably a human antibody, a human chimeric antibody, or a humanized antibody.

The term "antibody" typically means an immunoglobulin molecule, such as IgG, IgE, IgM, IgA, and IgD. In the present specification, the "antibody" includes an antibody fragment including an antigen binding region (e.g., F(ab')2 fragment, Fab' fragment, Fab fragment, Fv fragment, rIgG fragment, and a single chain antibody), and a modified antibody (e.g., a labeled antibody).

Examples of another embodiment of the protein include enzyme.

Examples of the enzyme include hydrolase, phosphorylase, dephosphorylase, transferase, oxidoreductase, lyase, isomerase, and synthesized enzyme.

Specific examples of the protein include quercetin, testosterone, indometacin, tranilast, and tacrolimus.

--Saccharides--

Examples of the saccharides include monosaccharide, disaccharide, oligosaccharide, and polysaccharide. Moreover, the saccharides also include complex carbohydrates, in which the saccharides are bonded to proteins or lipids via a covalent bond, and glycosides, in which aglycone, such as alcohol, phenol, saponin, and a dye, is bonded to a reducing group of saccharide.

--Lipids--

Examples of the liquids include simple lipids, complex lipids, and derived lipids.

--Low-molecular weight compound--

The low-molecular weight compound typically includes a natural synthetic substance having a molecular weight of from several hundreds through several thousands. The molecular weight may be a weight average molecular weight or a number average molecular weight.

As the low-molecular weight compound, moreover, there are a material equivalent to the above-mentioned poorly water-soluble material, and a material equivalent to the above-mentioned water-soluble material.

The poorly water-soluble material is a material having a water/octanol partition coefficient (logP value) of 3 or greater, as measured according to JIS Z 7260-107.

Moreover, the water-soluble material is a material having a water/octanol partition coefficient (logP value) of less than 3, as measured according to JIS Z 7260-107.

The low-molecular weight compound may be in the form of a salt, or hydrate, as long as the low-molecular weight compound functions as a physiologically active substance.

The poorly water-soluble material is not particularly limited and may be appropriately selected depending on the intended purpose. Examples of the poorly water-soluble compound include griseofulvin, ibuprofen, itraconazole, norfloxacin, tamoxifen, cyclosporine, glibenclamide, troglitazone, nifedipine, phenacetin, phenytoin, digitoxin, nilvadipine, diazepam, chloramphenicol, indomethacin, nimodipine, dihydroergotoxine, cortisone, dexamethasone, naproxen, tulobuterol, beclometasone dipropionate, fluticasone propionate, pranlukast, tranilast, loratadine, tacrolimus, amprenavir, bexarotene, calcitriol, clofazimine, digoxin, doxercalciferol, dronabinol, etopodide, isotretinoin, lopinavir, ritonavir, progesterone, saquinavir, sirolimus, tretinoin, amphotericin, fenoldopam, melphalan, paricalcitol, propofol, voriconazole, ziprasidone, docetaxel, haloperidol, lorazepam, teniposide, testosterone, valrubicin. The above-listed examples may be used alone or in combination.

Specific examples of the poorly water-soluble material include kinase inhibitors, such as gefitinib, erlotinib, osimertinib, bosutinib, vandetanib, alectinib, lorlatinib, abemaciclib, tyrphostin AG494, sorafenib, dasatinib, lapatinib, imatinib, motesanib, lestaurtinib, tandutinib, dorsomorphin, axitinib, 4-benzyl-2-methyl-1,2,4-thiadiazolidine-3,5-dione.

The water-soluble material is not particularly limited and may be appropriately selected depending on the intended purpose. Examples of the water-soluble material include abacavir, acetaminophen, acyclovir, amiloride, amitriptyline, antipyrine, atropine, buspirone, caffeine, captopril, chloroquine, chlorpheniramine, cyclophosphamide, diclofenac, desipramine, diazepam, diltiazem, diphenhydramine, disopyramide, doxine, doxycycline, enalapril, ephedrine, ethambutol, ethinylestradiol, fluoxetine, imipramine, glucose, ketorol, ketoprofen, labetalol, levodopa, levofloxacin, metoprolol, metronidazole, midazolam, minocycline, misoprostol, metformin, nifedipine, phenobarbital, prednisolone, promazine, propranolol, quinidine, rosiglitazone, salicylic acid, theophylline, valproic acid, verapamil, and zidovudine. The above-listed examples may be used alone or in combination.

-Physiologically active substance included in functional food product-

The physiologically active substance included in a functional food product is not particularly limited and may be appropriately selected depending on the intended purpose. Examples thereof include vitamin A, vitamin D, vitamin E, lutein, zeaxanthin, lipoic acid, flavonoid, and fatty acid. The above-listed examples may be used alone or in combination.

Examples of the fatty acid include omega-3 fatty acid and omega-6 fatty acid.

-Physiologically Active substance included in functional cosmetic product-

The physiologically active substance included in a functional cosmetic product is not particularly limited and may be appropriately selected depending on the intended purpose. Examples thereof include alcohols, aliphatic alcohols, polyols, fatty alcohols, and polyols, aldehydes, alkanol amines, alkoxylated alcohols (e.g., polyethylene glycol derivatives of alcohols or fatty alcohols), alkoxylated amides, alkoxylated amines, alkoxylated carboxylic acids, amides including salts of the amides (e.g., ceramides), amines, amino acids including salts and alkyl-substituted derivatives of the amino acids, esters, alkyl-substituted and acyl derivatives, polyacrylic acids, acrylamide copolymers, adipic acid copolymer aqueous solution, amino silicones, biological polymers and derivatives of the biological polymers, butylene copolymers, hydrocarbons (e.g., polysaccharides, chitosans, derivatives of the polysaccharides or chitosans), carboxylic acids, carbomers, esters, ethers, and polymeric ethers (e.g., PEG derivatives and PPG derivatives), glyceryl esters and derivatives of the glyceryl esters, halogen compounds, heterocyclic compounds including salts of the heterocyclic compounds, hydrophilic colloids and derivatives including salt and gum of the hydrophilic colloids (e.g., cellulose derivatives, gelatin, xanthan gum, natural rubber), imidazolines, inorganic materials (clay, $TiO_2$, ZnO), ketones (e.g., camphor), isethionates, lanolin and derivatives of the lanolin, organic salts, phenols including salts of the phenols (e.g., parabens), phosphorus compounds (e.g., phosphoric acid derivatives), polyacrylates and acrylate polymers, protein and enzyme derivatives (e.g., collagen), synthetic polymers including salts of the synthetic polymers, siloxanes and silanes, sorbitan derivatives, sterols, sulfonic acids and derivatives of the sulfonic acids, and wax. The above-listed examples may be used alone or in combination.

As described above, the physiologically active substance is preferably a substance bioactivity of which changes as a result of heating, cooling, or application of external stress. When such a physiologically active substance is included in the particles of the present disclosure, reduction in a level of bioactivity of the produced particles can be prevented. Accordingly, the effect of the present disclosure is significantly exhibited by using a physiologically active substance bioactivity of which is easily changed by heating, cooling, or application of external stress, as the physiologically active substance included in the particles of the present disclosure. Specifically, the physiologically active substance is preferably a physiologically active substance that can be included in a pharmaceutical composition, more preferably at least one selected from the group consisting of proteins and nucleic acids, and even more preferably at least one selected from the group consisting of antibodies and enzymes.

An amount of the physiologically active substance is preferably 1.0% by mass or greater but 50% by mass or less, and more preferably 5.0% by mass or greater but 40% by mass or less, relative to a total amount of the particles. When the amount of the physiologically active substance is 1.0% by mass or greater but 50% by mass or less relative to a total amount of the particles, the physiologically active substance can be encapsulated in the base material and thus the physiologically active substance can be gradually released.

The particles of the present disclosure can be used, for example, as a pharmaceutical composition, a functional food product, a functional cosmetic product etc., by optionally used in combination with other substances, such as a dispersant and additives. Moreover, the particles may be prepared as functional particles depending on various applications. The functional particles are not particularly limited and may be appropriately selected depending on the intended purpose. Examples thereof include immediate-release particles, sustained-release particles, pH-dependent-release particles, pH-independent-release particles, enteric-coated particles, controlled-release-coated particles, and nanocrystal-containing particles.

-Pharmaceutical composition-

The pharmaceutical composition includes the particles of the present disclosure, and may further includes additives, such as additives for pharmaceutical preparation, according to the necessity. The additives are not particularly limited and may be appropriately selected depending on the intended purpose. Examples of the additives include an excipient, a flavoring agent, a disintegrating agent, a fluidizer, an adsorbent, a lubricant, an odor-masking agent, a surfactant, a perfume, a colorant, an anti-oxidant, a masking agent, an anti-static agent, and a humectant. The above-listed examples may be used alone or in combination.

--Excipient--

The excipient is not particularly limited and may be appropriately selected depending on the intended purpose. Examples of the excipient include lactose, sucrose, mannitol, glucose, fructose, maltose, erythritol, maltitol, xylitol, palatinose, trehalose, sorbitol, microcrystalline cellulose, talc, silicic acid anhydride, anhydrous calcium phosphate, precipitated calcium carbonate, and calcium silicate. The above-listed examples may be used alone or in combination.

--Flavoring agent--

The flavoring agent is not particularly limited and may be appropriately selected depending on the intended purpose. Examples of the flavoring agent include L-menthol, refined sugar, D-sorbitol, xylitol, citric acid, ascorbic acid, tartaric acid, malic acid, aspartame, acesulfame potassium, thaumatin, saccharin sodium, dipotassium glycyrrhizinate, sodium glutamate, sodium 5'-inosinate, and sodium 5'-guanylate. The above-listed examples may be used alone or in combination.

--Disintegrating--

The disintegrating is not particularly limited and may be appropriately selected depending on the intended purpose. Examples of the disintegrating agent include hydroxypropyl celluloses with a low substitution degree, carmellose, carmellose calcium, carboxymethyl starch sodium, croscarmellose sodium, crospovidone, hydroxypropyl starch, and corn starch. The above-listed examples may be used alone or in combination.

--Fluidizer--

The fluidizer is not particularly limited and may be appropriately selected depending on the intended purpose.

Examples of the fluidizer include light anhydrous silicic acid, hydrated silicon dioxide, and talc. The above-listed examples may be used alone or in combination.

As the light anhydrous silicic acid, a commercial product can be used. The commercial product thereof is not particularly limited and may be appropriately selected depending on the intended purpose. Examples of the commercial product thereof include ADSOLIDER 101 (available from Freund Corporation, average pore diameter: 21 nm).

--Adsorbent--

As the adsorbent, a commercial product can be used. The commercial product of the adsorbent is not particularly limited and may be appropriately selected depending on the intended purpose. Examples thereof include product name: CARPLEX (component name: synthetic silica, registered trademark of DSL. Japan Co., Ltd.), product name: AEROSIL (registered trademark of NIPPON AEROSIL CO., LTD.) 200 (component name: hydrophilic fumed silica), product name: SYLYSIA (component name: amorphous silicon dioxide, registered trademark of Fuji Silysia Chemical Ltd), and product name: ALCAMAC (component name: synthetic hydrotalcite, registered trademark of Kyowa Chemical Industry Co., Ltd.). The above-listed examples may be used alone or in combination.

--Lubricant--

The lubricant is not particularly limited and may be appropriately selected depending on the intended purpose. Examples of the lubricant include magnesium stearate, calcium stearate, sucrose fatty acid ester, sodium stearyl fumarate, stearic acid, polyethylene glycol, and talc. The above-listed examples may be used alone or in combination.

--Odor-masking agent--

The odor-masking agent is not particularly limited and may be appropriately selected depending on the intended purpose. Examples of the odor-masking agent include trehalose, malic acid, maltose, potassium gluconate, aniseed essential oil, vanilla essential oil, and cardamom oil. The above-listed examples may be used alone or in combination.

--Surfactant--

The surfactant is not particularly limited and may be appropriately selected depending on the intended purpose. Examples of the surfactant include: polysorbate such as polysorbate 80; a polyoxyethylene-polyoxypropylene copolymer; and sodium lauryl sulfate. The above-listed examples may be used alone or in combination.

--Perfume--

The perfume is not particularly limited and may be appropriately selected depending on the intended purpose. Examples of the perfume include lemon oil, orange oil, and peppermint oil. The above-listed examples may be used alone or in combination.

--Colorant--

The colorant is not particularly limited and may be appropriately selected depending on the intended purpose. Examples of the colorant include titanium oxide, Food Yellow No. 5, Food Blue No. 2, iron sesquioxide, and yellow iron sesquioxide. The above-listed examples may be used alone or in combination.

--Anti-oxidant--

The anti-oxidant is not particularly limited and may be appropriately selected depending on the intended purpose. Examples of the anti-oxidant include sodium ascorbate, L-cysteine, sodium sulfite, and vitamin E. The above-listed examples may be used alone or in combination.

--Masking agent--

The masking agent is not particularly limited and may be appropriately selected depending on the intended purpose. Examples of the masking agent include titanium oxide. The above-listed examples may be used alone or in combination.

--Anti-static agent--

The anti-static agent is not particularly limited and may be appropriately selected depending on the intended purpose. Examples of the anti-static agent include talc and titanium oxide. The above-listed examples may be used alone or in combination.

--Humectant--

The humectant is not particularly limited and may be appropriately selected depending on the intended purpose. Examples of the humectant include polysorbate 80, sodium lauryl sulfate, sucrose fatty acid ester, macrogol, and hydroxypropyl cellulose (HPC). The above-listed examples may be used alone or in combination.

A pharmaceutical preparation of the pharmaceutical composition is not particularly limited and may be appropriately selected depending on the intended purpose. Examples thereof include colon delivery preparations, lipid microsphere preparations, dry emulsion preparations, self-emulsifying preparations, dry syrup, powder preparations for transnasal administration, powder preparations for pulmonary administration, wax matrix preparations, hydrogel preparations, polymeric micelle preparations, mucoadhesive preparations, gastric floating preparations, liposome preparations, and solid dispersion preparations. The above-listed examples may be used alone or in combination.

Examples of the dosage form of the pharmaceutical composition include: tablets, capsules, suppository, and other solid dosage forms; intranasal aerosol and aerosol for pulmonary administration; and liquid medicaments, such as injections, intraocular preparations, endaural preparations, and oral preparations.

The administration route of the pharmaceutical composition is not particularly limited and may be appropriately selected depending on the intended purpose. Examples thereof include oral administration, nasal administration, rectal administration, vaginal administration, subcutaneous administration, intravenous administration, and pulmonary administration.

(Method for producing sustained-release particles and apparatus for producing sustained-release particles)

The method for producing sustained-release particles of the present disclosure include: a droplet-ejecting step including ejecting droplets each including a physiologically active substance having bioactivity, a base material, and a solvent; and a granulating step including removing the solvent from the droplets to granulate particles. The method may further include other steps according to the necessity.

The apparatus for producing sustained-release particles of the present disclosure include: a droplet-ejecting unit configured to eject droplets each including bioactivity, a base material, and a solvent; and a granulating unit configured to remove the solvent from the droplets to granulate particles. The apparatus may further include other units according to the necessity.

In the present specification, the term "removing" means removing the solvent included in the liquid phase. However, the removing is not limited to removing all of the solvent included in the liquid phase, but also includes a case where some of the solvent included in the liquid phase is remained in the liquid phase, as long as particles can be granulated. In the present specification, moreover, the embodiment of the term "removing" is not particularly limited as long as the solvent included in the liquid phase can be removed. Examples thereof include: an embodiment where the liquid phase is brought into contact with another liquid phase to disperse the solvent included in the liquid phase into the above-mentioned another liquid phase (this embodiment may be referred to as "liquid drying" hereinafter); and an embodiment where the solvent included in the liquid phase is evaporated from the liquid phase in gas or vacuumed atmosphere (this embodiment may be referred to as "gas drying" hereinafter).

Next, an example where removing is performed by liquid drying will be described as a first embodiment, and an example where removing is performed by gas drying will be described as a second embodiment. However, the method and apparatus for producing particles are not limited to the first and second embodiments.

[First embodiment (liquid drying)]

<Method for producing particles (first embodiment)>

A method for producing particles according to the first embodiment (liquid drying) includes a droplet-ejecting step and a granulating step, and may further include other steps according to the necessity. The droplet-ejecting step includes ejecting droplets each including a physiologically active substance having bioactivity, a base material, and a good solvent to the base material into a poor solvent to the base material. The granulating step includes allowing the droplets to be in contact with the poor solvent to remove the good solvent included in the droplets to granulate particles.

Various methods have been known in the art as a wet granulation method where particles are granulated in a liquid, as in the first embodiment.

Examples thereof include a wet pulverization method where particles material in a liquid are stirred with high shear force using a media stirrer or a media-less stirrer, to obtain pulverized particles having small particle diameters.

Moreover, examples thereof include: a method where a good solvent, in which particle materials are dissolved, is added to a poor solvent stirred with a high shearing force using mixing stirrer, to thereby obtain precipitated particles having small particle diameters; and a two-liquid mixing method where a liquid including particle materials and a solvent that dissolves the particle materials is added to an aqueous medium in the presence of a surfactant, while stirring the aqueous medium with high shear force using a mixing stirrer, to thereby obtain dispersed particles having small particle diameters.

Furthermore, examples thereof include an emulsion method where the components, such as the physiologically active substance, and the base materials, are dissolved in respectively different two or more solutions, the resultant solutions are emulsified, and the solvent is evaporated from the emulsion to obtain particles, in each of which the physiologically active substance is encapsulated in the base material.

The wet pulverization can produce particles of small particle diameters, but it is difficult to produce particles having a narrow particle size distribution.

When the media stirrer is used, moreover, the resultant particles may include impurities derived from the media. Therefore, it may be difficult to add such particles to a pharmaceutical composition, a functional food product, a functional cosmetic product, etc.

When the media-less stirrer is used, moreover, productivity may be low.

In the wet pulverization method, large external stress is generated by stirring with high shear force during the pulverization process thereof. In the case where the physiologically active substance whose bioactivity changes by application of the external stress is included as a material of the particles, therefore, bioactivity of the physiologically active substance changes, and as a result, the level of bioactivity reduces.

The two-liquid mixing method can produce particles having a narrow particle size distribution, but a surfactant for use may be remained in the produced particles. Depending on the surfactant for use, the resultant particles may not be able to add to a pharmaceutical composition, a functional food product, a functional cosmetic product, etc.

It is possible to perform a treatment for removing the physiologically active substance or the surfactant from the particles. In the case where the bioactivity of the physiologically active substance changes on heating, cooling, or application of external stress, the bioactivity may be changed by the treatment, and as a result, the level of the bioactivity may reduce.

In the two-liquid mixing method, large external stress is generated by stirring with high shear force during the mixing and stirring process thereof. In the case where the physiologically active substance whose bioactivity changes by application of the external stress is included as a material of the particles, therefore, bioactivity of the physiologically active substance changes, and as a result, the level of bioactivity reduces.

The emulsion method can produce particles having a narrow particle size distribution, but it is difficult to encapsulate all of the added physiologically active substance in the base material. Therefore, some of the physiologically active substance added is wasted.

The method for producing particles according to the first embodiment, which will be described hereinafter, is not an equivalent of the wet pulverization or two-liquid mixing. Even in the case where a physiologically active substance whose bioactivity changes by heating, cooling, or application of external stress is included as a material of the particles, therefore, a change in the bioactivity of the physiologically active substance is prevented, and as a result, the level of bioactivity is not lowered.

As described hereinafter, moreover, the method for producing particles of the first embodiment preferably does not include a step using a member for shaking or stirring to impart a degree of external stress by which bioactivity of the physiologically active substance changes, a step using a member for heating to a temperature at which the bioactivity of the physiologically active substance changes, and a step using a member for cooling to a temperature at which the bioactivity of the physiologically active substance changes.

-Droplet-ejecting step (first embodiment)-

In the first embodiment, the droplet-ejecting step is a step including ejecting droplets each including a physiologically active substance having bioactivity, a base material, and a good solvent to the base material into a poor solvent.

A method for ejecting droplets is not particularly limited. Examples thereof include the following methods.

(i) A method using an ejection unit where pressure is applied to the liquid to eject the liquid as droplets from pores made in a flat surface to which nozzles are formed, such as inkjet nozzles.
(ii) A method using an ejection unit where pressure is applied to the liquid to eject the liquid as droplets from pores of irregular shapes, such as a SPG film.

(iii) A method using an ejection unit where vibrations are applied to the liquid to eject the liquid as droplets from pores.

Examples of the ejection unit using the vibrations as mentioned in (iii) above, which is a unit that does not change bioactivity of the physiologically active substance due to the vibration, include units that do not easily apply external stress to the particle composition liquid itself, such as an ejection unit using a membrane vibration method, an ejection unit using the Rayleigh breakup method, an ejection unit using a liquid vibration method, and an ejection unit using a liquid column resonance method. Moreover, the above-listed ejection units may further include a unit configured to eject the liquid as pressure is applied to the liquid. Among the above-listed examples, an ejection unit that is the ejection unit using the liquid column resonance method and includes a unit configured to apply pressure to the liquid to eject the liquid is preferable.

Examples of the ejection unit using the liquid column resonance method include an ejection unit using a method where vibrations are applied to the liquid stored in a liquid column resonance liquid chamber to form standing waves due to liquid column resonance to eject the liquid from an ejection hole formed in the amplification direction of the standing waves in the regions corresponding to anti-nodes of the standing waves.

Examples of the ejection unit using the membrane vibration method include the ejection units disclosed in Japanese Unexamined Patent Application Publication No. 2008-292976.

Examples of the ejection unit using the Rayleigh breakup method include the ejection units disclosed in Japanese Patent No. 4647506.

Examples of the ejection unit using the liquid vibration method include the ejection units disclosed in Japanese Unexamined Patent Application Publication No. 2010-102195.

The ejection hole formed in the ejection unit and from which droplets are ejected has a diameter of preferably smaller than 1,000 $\mu$m, more preferably 1.0 $\mu$m or greater but smaller than 1,000 $\mu$m, even more preferably 1.0 $\mu$m or greater but 500 $\mu$m or smaller, and particularly preferably 1.0 $\mu$m or greater but 50 $\mu$m or smaller. When the shape of the ejection hole is not a true circle, a diameter of a true circle having the same area to the area of the ejection hole is determined as the diameter of the ejection hole.

The droplet-ejecting step may be performed in the state where the ejection hole is placed in the poor solvent (in other words, a state where the ejection hole is in contact with the poor solvent), or in the state where the ejection hole is placed outside the poor solvent (in other words, a state where the ejection hole is not in contact with the poor solvent). The droplet-ejecting step is preferably performed in the state where the ejection hole is placed in the poor solvent. Since the ejection of the liquid is performed in the state where the ejection hole is placed in the poor solvent, the liquid ejected from the ejection hole is prevented from being dried, and ejection failures can be prevented. Moreover, the particle size distribution of the produced particles can be made small.

When the ejection hole is placed in the poor solvent, a length between a liquid surface of the poor solvent and the ejection hole (in other words, the depth of the position of the ejection hole set in the poor solvent) is not particularly limited and may be appropriately selected depending on the intended purpose. The length is preferably 1.0 mm or greater but 10 mm or less, and more preferably 2.0 mm or greater but 5.0 mm or less.

In the droplet-ejecting step, the liquid to be ejected (i.e., the particle composition liquid) includes a base material, a physiologically active substance having bioactivity, and a good solvent to the base material, and the liquid is ejected into a poor solvent to the base material. Since any of the various materials listed as the base material and physiologically active substance included in the particles can be used as the base material and physiologically active substance included in the liquid, descriptions thereof are omitted here and only the good solvent and the poor solvent will be described hereinafter.

The particle composition liquid and the poor solvent are preferably substantially free from a surfactant. Since the particle composition liquid and poor solvent are substantially free from a surfactant, safety is improved when the produced particles are added to a pharmaceutical composition, a functional food product, a functional cosmetic product etc. In the present specification, the phrase "substantially free from a surfactant" means, for example, a case where an amount of a surfactant in the particle composition liquid, and the amount thereof in the poor solvent are equal to or below the detection limit, below which the amount thereof cannot be detected by liquid chromatography, or a case where a surfactant is not included in the particle composition liquid and the poor solvent.

--Good solvent--

In the present specification, the term "good solvent" means a solvent with which solubility of the base material is high. Moreover, the good solvent can be defined by a mass of the base material that can be dissolved with 100 g of the solvent at a temperature of 25°C. As the good solvent, for example, a mass of the base material that can be dissolved in the solvent is preferably 0.10 g or greater.

The good solvent is also preferably a solvent with which solubility of the physiologically active substance is high. Similarly to the base material, for example, a mass of the physiologically active substance that can be dissolved in the solvent is preferably 0.10 g or greater.

The good solvent is not particularly limited and may be appropriately selected depending on the intended purpose. Examples of the good solvent include alcohol, ketone, ether, acetonitrile, and tetrahydrofuran. Examples of alcohol include C1-C4 alcohol. Examples of C1-C4 alcohol include methanol, ethanol, propanol, and butanol. Examples of ketone include C3-C6 ketone. Examples of C3-C6 ketone include acetone, methyl ethyl ketone, and cyclohexanone. Examples of ether include C2-C6 ether. Examples of C2-C6 ether include dimethyl ether, methyl ethyl ether, and diethyl ether. The above-listed examples may be used alone or in combination. Among the above-listed examples, a good solvent including alcohol and ketone in combination is preferable, and a good solvent including ethanol and acetone in combination is more preferable.

An amount of the base material in the particle composition liquid is not particularly limited and may be appropriately selected depending on the intended purpose. In the case where acetone and ethanol are used in combination as a good solvent, for example, the amount of the base material is preferably 5.0% by mass or less, and more preferably 0.1% by mass or greater but 5.0% by mass or less, relative to a mass of the particle composition liquid. When the amount of the base material in the particle composition liquid is 5.0% by mass or less, the particle size distribution of the particles can be made narrow. The number average particle diameter of the produced particles can be controlled by adjusting the amount of the base material.

--Poor solvent--

In the present specification, the term "poor solvent" means a solvent with which solubility of the base material is low, or in which the base material is not dissolved. The poor solvent can be defined by a mass of the base material that can be dissolved with 100 g of the solvent at a temperature of 25°C. As the poor solvent, for example, a mass of the base material that can be dissolved in the solvent is preferably 0.05 g or less.

The poor solvent is also preferably a solvent with which solubility of the physiologically active substance is low. Similarly to the base material, for example, a mass of the physiologically active substance that can be dissolved in the solvent is preferably 0.05 g or less.

The poor solvent is not particularly limited and may be appropriately selected depending on the intended purpose. For example, the poor solvent is preferably water.

A stabilizer may be added to the poor solvent for the purpose of suppressing aggregations of produced particles, or crystal growth of the physiologically active substance.

The stabilizer is not particularly limited and may be appropriately selected depending on the intended purpose. Examples of the stabilizer include polyethylene glycol fatty acid ester, sorbitan fatty acid ester, polyoxyethylene hydrogenated castor oil, polyoxyethylene alkyl ether, quaternary ammonium salt, lecithin, polyvinyl pyrrolidone, polyvinyl alcohol, glyceride, fatty acid, and steroid. Among the above-listed examples, polyethylene glycol fatty acid ester, sorbitan fatty acid ester, polyvinyl pyrrolidone, polyvinyl alcohol, glyceride, fatty acid, steroid, and phospholipid are preferable. Moreover, polyethylene glycol fatty acid ester, sorbitan fatty acid ester, and fatty acid are more preferable. Specifically, polyoxyl 40 stearate, polysorbate 80, and stearic acid are preferable. The above-listed examples may be used alone or in combination.

Moreover, an amount of the stabilizer in the poor solvent is preferably 5% by mass or less relative to a mass of the poor solvent.

Since the poor solvent includes the stabilizer, surfaces of particles formed in the poor solvent are covered with the stabilizer to form a hydrophilic coating layer on each particle. Therefore, the particles are easily absorbed in vivo. The coating may completely cover a particle, or may partially cover a particle.

-Granulating step (first embodiment)-

In the first embodiment, the granulating step is a step including allowing the droplets to be in contact with the poor solvent to remove the good solvent included in the droplets to granulate particles.

Specifically, it is a step where the droplets ejected into the poor solvent in the droplet-ejecting step are allowed to be in contact with the poor solvent to cause interdiffusion between the good solvent included in the droplets and the poor solvent, and the base material included in the droplets is supersaturated, thus the base material is precipitated to granulate particles.

Since the particles are granulated in the step as described above, the particles can be obtained as solid dispersions. Specifically, the particles, in each of which the physiologically active substance is dispersed in the base material, can be produced.

The particles produced by the above-described granulating step are granulated with maintaining shapes of the droplets as the particle material liquid in the form of droplets is brought into contact with the poor solvent. Therefore, a narrow particle size distribution of the particles can be achieved by stably forming droplets of a unified size, compared with particles produced by any other methods known in the art.

Moreover, the particle diameters of the particles can be adjusted by appropriately adjusting a size of the ejection hole of the ejection unit configured to form droplets.

Even in the case where the physiologically active substance whose bioactivity changes by application of external stress is included as a material of the particles, moreover, a change in the bioactivity of the physiologically active substance is prevented by forming droplets with the ejecting unit to make the particle diameters of the particles small, instead of using a stirrer for stirring with high shear force. As a result, the level of the bioactivity is not lowered.

Accordingly, the bioactivity rate of the particles can be improved by the method as described above, compared with the methods known in the art. For example, the bioactivity rate can be improved to 50% or greater.

It is preferred that the poor solvent be made flown in the granulating step.

The flow rate of the poor solvent is not particularly limited as long as strong external stress, which may affect bioactivity of the physiologically active substance, is not generated. For example, the flow rate of the poor solvent is preferably 0.3 m/s or greater, and more preferably 1.0 m/s or greater. Since the poor solvent is made flown, cohesion between particles can be prevented, and thus a narrow particle size distribution of the particles is obtained.

It is preferred that the poor solvent be circulated in the granulating step. The circulation is preferably performed by providing a circulation path in the poor solvent storage contained in which the poor solvent is stored. Since the poor solvent is circulated, cohesion between particles can be prevented, and thus a narrow particle size distribution of the particles is obtained.

The granulating step preferably includes removing the good solvent accumulated in the poor solvent as the droplets are ejected into the poor solvent. Since the good solvent in the poor solvent is removed, cohesion between particles can be prevented, and thus a narrow particle size distribution of the particles is obtained.

-Other steps-

Examples of other steps include a good solvent removing step, a filtration sterilization step, and a poor solvent removing step.

--Good solvent removing step--

The good solvent removing step is not particularly limited and may be appropriately selected depending on the intended purpose, as long as the good solvent removing step is a step including removing the good solvent from the liquid including the produced particles. Examples thereof include a method where a decompression treatment is performed on the liquid including the particles to evaporate the good solvent to obtain a suspension liquid including the particles.

--Filtration sterilization step--

The filtration sterilization step is not particularly limited and may be appropriately selected depending on the intended purpose, as long as the filtration sterilization step is a step including performing filtration of the particle suspension liquid after the good solvent removing step using a sterilization filter. Moreover, the suspension liquid including particles, which is provided to the filtration, may be diluted or may not be diluted with a poor solvent.

The sterilization filter is not particularly limited and may be appropriately selected depending on the intended purpose. Examples thereof include a nylon membrane filter. The filtration rating of the sterilization filter is not particularly limited and may be appropriately selected depending on the intended purpose. The filtration rating thereof is preferably 0.1 μm or greater but 0.45 μm or less. As the sterilization filter, a commercial product may be used. Examples of the commercial product include LifeASSUR™ nylon membrane filter cartridge (filtration rating: 0.1 μm).

--Poor solvent removing step--

The poor solvent removing step is not particularly limited and may be appropriately selected depending on the intended purpose, as long as the poor solvent removing step is a step including removing the poor solvent from the filtrate after the sterilization filtration. Examples thereof include a method where the poor solvent is removed by filtration.

Other than the steps mentioned above, examples of the above-described other steps include a step where, after granulation of particles, the particles are passed through a sieve to obtain particles of a uniform particle size.

<Apparatus for producing particles (first embodiment)>

The apparatus for producing particle according to the first embodiment (liquid drying) includes a droplet-ejecting unit and a granulating unit. The droplet-ejecting unit is configured to eject droplets, each including a physiologically active substance having bioactivity, a base material, and a good solvent to the base material, into a poor solvent to the base material. The granulating unit is configured to allow the droplets into contact with the poor solvent to remove the good solvent inside the droplets to thereby granulate particles. The apparatus may further include other units according to the necessity.

-Droplet-ejecting unit (first embodiment)-

The droplet-ejecting unit is a unit configured to eject droplets, each including a physiologically active substance, a base material, and a good solvent to the base material, into a poor solvent to the base material.

The droplet-ejecting unit is connected to the below-described liquid storage container. A member for connecting between the droplet-ejecting unit and the liquid storage container is not particularly limited and may be appropriately selected depending on the intended purpose, as long as such a member can supply the liquid from the liquid storage container to the droplet-ejecting unit. Examples thereof include pipes and tubes.

The droplet-ejecting unit is not particularly limited as long as the droplet-ejecting unit is capable of ejecting droplets. The droplet-ejecting unit preferably includes a vibration applying member configured to apply vibrations to the liquid to eject the liquid in the form of droplets. The vibrations are not particularly limited and may be appropriately selected depending on the intended purpose. For example, the frequency is preferably 1 kHz or greater, more preferably 150 kHz or greater, and even more preferably 300 kHz or greater but 500 kHz or less. When the vibrations are 1 kHz or greater, liquid columns ejected from the ejection holes can be formed into droplets with good reproducibility. When the vibrations are 150 kHz or greater, production efficiency can be improved.

Examples of the droplet-ejecting unit including the vibration applying member include inkjet nozzles. As the ejection mechanism of the inkjet nozzles, for example, a liquid column resonance method, a membrane vibration method, a liquid vibration method, a Rayleigh breakup method, etc. may be used.

--Liquid storage container--

The liquid storage container is a container, in which the liquid including the base material, the physiologically active substance, and the good solvent is stored.

The liquid storage container may be or may not be flexible. A material of the liquid storage container is not particularly limited and may be appropriately selected depending on the intended purpose. The material thereof may be a resin or a metal. A structure of the liquid storage container is not particularly limited and may be appropriately selected depending on the intended purpose. Examples of the structure thereof include a sealed structure or a non-sealed structure.

-Granulating unit (first embodiment)-

The granulating unit is a unit configured to allow the droplets to be in contact with a poor solvent to remove the good solvent inside the droplets to granulate particles.

Examples of the granulating unit include a poor solvent storage container, in which the poor solvent is stored. The poor solvent storage container may be or may not be flexible. A material of the poor solvent storage container is not particularly limited and may be appropriately selected depending on the intended purpose. For example, the material thereof may be a resin or a metal.

The granulating unit preferably includes a flow unit configured to make the poor solvent flow.

The granulating unit preferably has a circulation path to which a circulating unit is disposed, and the circulating unit is configured to circulate the stored poor solvent. For example, the circulation path may be a circulation path composed only of piping, or a circulation path including piping and a tank.

The granulating unit preferably includes a good solvent removing configured to remove the good solvent accumulated in the poor solvent as droplets are ejected into the poor solvent. Examples of the good solvent removing unit include a compression unit.

Next, a specific embodiment of the first embodiment will be described based on an embodiment where a liquid column resonance droplet-ejecting unit is used as the droplet-ejecting unit.

It is easily understood by a person skilled in the art that the droplet-ejecting unit is not limited to the liquid column resonance droplet-ejecting unit, and other droplet-ejecting units (e.g., an ejection unit using a membrane vibration method, an ejection unit using the Rayleigh breakup method, and an ejection unit using a liquid vibration method) may be used.

First, the liquid column resonance droplet-ejecting unit, which is one element constituting the apparatus for producing particles, will be specifically described.

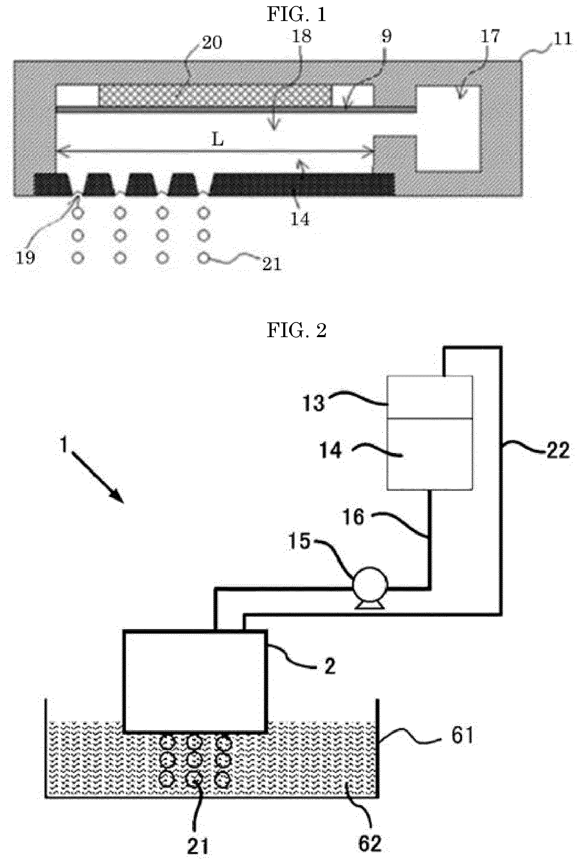

FIG. 1 is a schematic cross-sectional view illustrating one example of the liquid column resonance droplet-ejecting unit. The liquid column resonance droplet-ejecting unit 11 includes a common liquid supplying path 17 and a liquid-column-resonance liquid chamber 18. The liquid-column-resonance liquid chamber 18 is connected to the common liquid supplying path 17 disposed on one of wall surfaces at both ends in a longitudinal direction. Moreover, the liquid-column-resonance liquid chamber 18 includes an ejection hole 19 and a vibration generating unit 20. The ejection hole 19 is configured to eject liquid droplets 21 and arranged on one of the wall surfaces connected to the wall surfaces at the both ends. The vibration generating unit 20 is configured to generate high frequency vibrations to form liquid column resonance standing waves. Note that, a high frequency power source, which is not illustrated, is coupled to the vibration generating unit 20. Moreover, a flow channel may be disposed. The flow channel is configured to supply a gas flow for transporting liquid droplets 21 ejected from the liquid column resonance ejecting unit 11.

The liquid 14 including the base material, the physiologically active substance, and the good solvent is passed through a liquid supply pipe and introduced into the common liquid supplying path 17 of the liquid column resonance liquid droplet forming unit 11 by a liquid-circulating pump that is not illustrated, and then is supplied to the liquid-column-resonance liquid chamber 18 of the liquid column resonance droplet-ejecting unit 11.

Within the liquid-column-resonance liquid chamber 18 charged with the liquid 14, a pressure distribution is formed by liquid column resonance standing waves generated by the vibration generating unit 20. Then, liquid droplets 21 are ejected from the ejection hole 19 disposed in the regions that correspond to anti-nodes of the standing waves where the regions are the sections where the amplitude of the liquid column resonance standing waves is large and pressure displacement is large.

The regions corresponding to anti-nodes of the standing waves owing to the liquid column resonance are regions other than nodes of the standing waves. The regions are preferably regions each having sufficiently large amplitude enough to eject the liquid through the pressure displacement of the standing waves, are more preferably regions having a width corresponding to ± 1/4 of a wavelength from a position of a local maximum amplitude of a pressure standing wave (i.e., a node of a velocity standing wave) toward positions of a local minimum amplitude.

Even when there are a plurality of openings of the ejection hole, substantially uniform droplets can be formed from the openings as long as the openings of the ejection hole are disposed in the regions corresponding to the anti-nodes of the standing waves. Moreover, ejection of the liquid droplets can be performed efficiently, and clogging of the ejection hole is unlikely to occur. Note that, the liquid 14 passed through the common liquid supplying path 17 travels through a liquid returning pipe (not illustrated) to be returned to the liquid 14. Once the amount of the liquid 14 inside the liquid-column-resonance liquid chamber 18 is reduced by ejection of the droplets 21, a flow rate of the liquid 14 supplied from the column liquid supplying path 17 by suction power generated by the action of the liquid column resonance standing waves inside the liquid-column-resonance liquid chamber 18. As a result, the liquid-column-resonance liquid chamber 18 is refilled with the liquid 14. When the liquid-column-resonance liquid chamber 18 is refilled with the liquid 14, the flow rate of the liquid 14 passed through the common liquid supplying path 17 returns to the previous flow rate.

The liquid-column-resonance liquid chamber 18 of the liquid column resonance droplet-ejecting unit 11 is formed by joining frames with each other. The frames are formed of materials having high stiffness to the extent that a resonance frequency of the liquid is not influenced at a driving frequency (e.g., metals, ceramics, and silicones).

As illustrated in FIG. 1, a length L between the wall surfaces at the both ends of the liquid-column-resonance liquid chamber 18 in a longitudinal direction is determined based on the principle of the liquid column resonance described below.

Moreover, a plurality of the liquid-column-resonance liquid chambers 18 are preferably disposed per one droplet forming unit in order to drastically improve productivity.

The number of the liquid-column-resonance liquid chambers 18 is not particularly limited. The number thereof is preferably 1 or greater but 2,000 or less.

The common liquid supplying-path 17 is coupled to and connected to a path for supplying the liquid for each liquid-column-resonance liquid chamber. The common liquid supplying path 17 is connected to a plurality of the liquid-column-resonance liquid chambers 18.

Moreover, the vibration generating unit 20 of the liquid column resonance droplet-ejecting unit 11 is not particularly limited as long as the vibration generating unit 20 is driven at a predetermined frequency. The vibration generating unit is preferably formed by attaching a piezoelectric material onto an elastic plate 9.

The frequency is preferably 150 kHz or greater, more preferably 300 kHz or greater but 500 kHz or less from the viewpoint of productivity.

The elastic plate constitutes a portion of the wall of the liquid-column-resonance liquid chamber in a manner that the piezoelectric material does not come into contact with the liquid.

The piezoelectric material may be, for example, piezoelectric ceramics such as lead zirconate titanate (PZT), and is typically often laminated due to a small displacement amount. Other examples of the piezoelectric material include piezoelectric polymers (e.g., polyvinylidene fluoride (PVDF)) and monocrystals (e.g., crystal, $LiNbO_3$, $LiTaO_3$, and $KNbO_3$).

The vibration generating unit 20 is preferably disposed per liquid-column-resonance liquid chamber in a manner that the vibration generating unit 20 can individually control each liquid-column-resonance liquid chamber.

It is preferable that the liquid-column-resonance liquid chambers be individually controlled via the elastic plates by partially cutting a block-shaped vibration member, which is formed of one of the above-described materials, according to geometry of the liquid-column-resonance liquid chambers.

Moreover, a plurality of openings are formed in the ejection hole 19. In view of productivity, preferably employed is a structure where the ejection hole 19 is disposed along the width direction of the liquid-column-resonance liquid chamber 18.

Moreover, the frequency of the liquid column resonance is desirably appropriately determined with checking ejection of droplets, because the frequency of the liquid column resonance varies depending on the arrangement of opening of the ejection hole 19.

Next, the apparatus for producing particles will be specifically described.

FIG. 2 is a schematic view illustrating one example of the apparatus for producing particles. The apparatus for producing particles 1 includes a liquid storage container 13, a droplet-ejecting unit 2, and a poor solvent storage container 61. To the droplet-ejecting unit 2, the liquid storage container 13, in which the liquid 14 is stored, and a liquid-circulating pump 15 are connected. The liquid-circulating pump 15 is configured to supply the liquid 14 stored in the liquid storage container 13 to the droplet-ejecting unit 2 via the liquid supply tube 16. Moreover, the liquid-circulating pump 15 is configured to pressure feed the liquid inside the liquid supply tube 16 to return to the liquid storage container 13 via a liquid returning tube 22. Therefore, the liquid 14 can be supplied to the droplet-ejecting unit 2 as needed.

For example, the droplet-ejecting unit 2 includes the liquid column resonance droplet-ejecting unit 11 illustrated in FIG. 1.

The liquid 14 is ejected as droplets 21 into the poor solvent 62 from the droplet-ejecting unit 2, where the poor solvent 62 is stored in the poor solvent storage container 61.

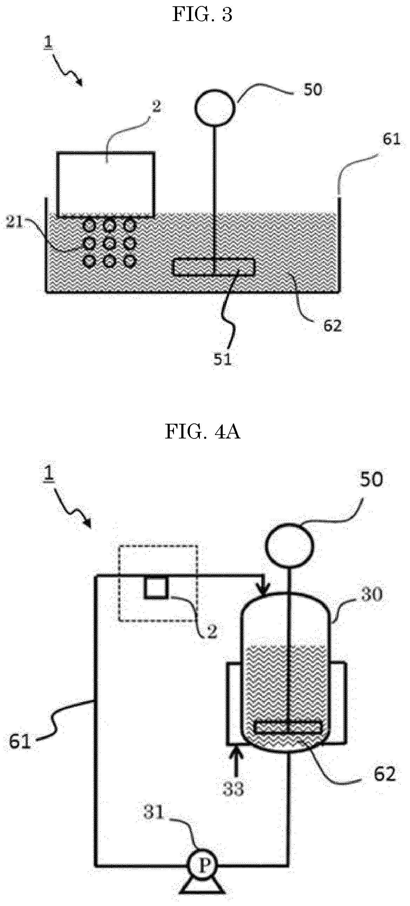

FIG. 3 is a schematic view illustrating another example of the apparatus for producing particles.

FIG. 3 depicts a schematic view of the apparatus 1 for producing particles where a liquid is ejected into a poor solvent 62 inside a poor solvent storage container 61 that is a glass container. An ejection part of the droplet-ejecting unit 2 is configured to eject the liquid into the poor solvent 62 in the state where the ejection part is immersed in the poor solvent 62.

The apparatus 1 for producing particles of FIG. 3 includes a stirring member 50 having a stirring blade 51. The stirring blade 51 is immersed in the poor solvent 62 inside the poor solvent storage container 61.

When the droplets 21 are ejected into the poor solvent 62 by the droplet-ejecting unit 2, the stirring blade 51 is rotated to stir the poor solvent 61. As a result, cohesion between particles formed from the droplets 21 can be prevented. The stirring by the stirring member 50 is performed not to change bioactivity of the physiologically active substance included in the particles. Moreover, the stirring may not be performed.

Next, another example of the apparatus for producing particles will be described.

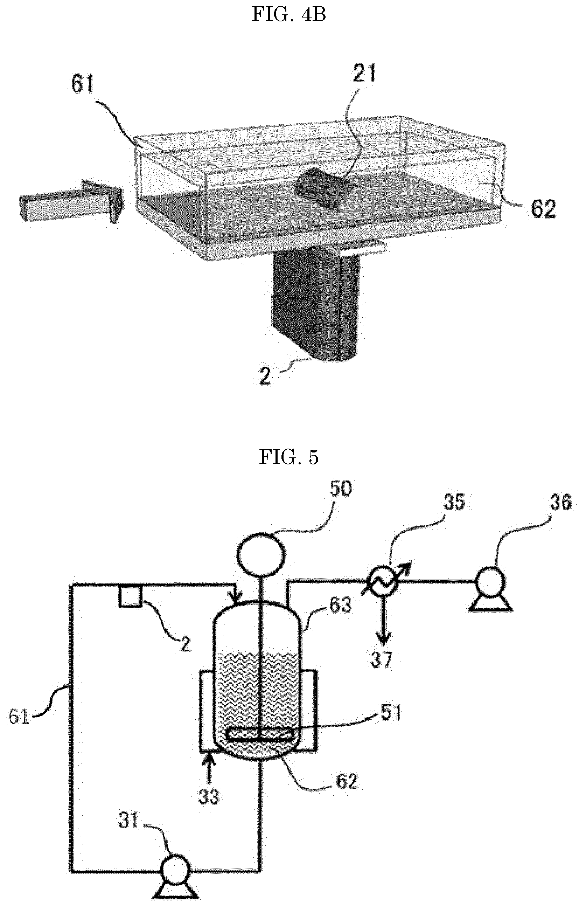

As a method for preventing cohesion between particles formed by allowing the droplets to be in contact with the poor solvent, for example, preferred is imparting a flow of the poor solvent to the ejection part of the droplet-ejecting unit. The method thereof will be described with reference to FIGs. 4A and 4B.

FIG. 4A is a schematic view illustrating one example of the apparatus for producing particles, where the apparatus can apply a flow of the poor solvent to the ejection part of the droplet-ejecting unit.

The apparatus for producing particles of FIG. 4A includes a droplet-ejecting unit 2, a poor solvent storage container 61, a stirring member 50, and a pump 31.

The poor solvent storage container 61 includes a circulation path capable of circulating the liquid. As part of the poor solvent storage container 61, a tank 30 is disposed in the middle of the circulation path.

FIG. 4B is an enlarged view of a section adjacent to the droplet-ejection unit 2 (section marked with a dashed line) of FIG. 4A.

The poor solvent 62 in the tank 30 is circulated inside the poor solvent storage container 61 via the droplet ejecting unit 2 by the pump 31. During the circulation, the liquid is ejected into the poor solvent 62 from the ejection hole of the droplet-ejecting unit 2. By applying a flow to the liquid that is the poor solvent 62, cohesion of particles formed from the droplets 21 can be prevented.

The tank 30 includes the stirring member 50 having a stirring blade. Cohesion of particles can be prevented by stirring the liquid that is the poor solvent 62 by the stirring blade.

Moreover, the tank 30 includes a heating unit 33 for removing the good solvent included in the poor solvent 62. The heating by the heating unit 33 is performed no to change the bioactivity of the physiologically active substance included in the particles. Moreover, the heating unit may not be included.

Next, another example of the apparatus for producing particles will be described.

As an amount of the good solvent increases in the poor solvent, cohesion of particles tends to occur to increase diameters of the particles. As a method for preventing the cohesion of the particles, it is preferred that the good solvent be removed from the poor solvent to keep the amount of the good solvent in the poor solvent low. The method thereof will be described with reference to FIG. 5.

FIG. 5 is a schematic view illustrating an example of the apparatus for producing particles, which includes a good solvent removing unit configured to remove a good solvent.

The apparatus for producing particles of FIG. 5 includes a droplet-ejecting unit 2, a poor solvent storage container 61, a stirring member 50, and a pump 31, and as a good solvent removing unit, a heating unit 33 and a decompression unit 36 (e.g., a vacuum pump).

The structure of the section adjacent to the droplet-ejecting unit 2 is identical to the structure illustrated in FIGs. 4A and 4B.

The poor solvent storage container 61 includes a circulation path capable of circulating the liquid. As part of the poor solvent storage container 61, a tank 63 is disposed in the middle of the circulation path.

The poor solvent 62 in the tank 63 is circulated inside the poor solvent storage container 61 via the droplet ejecting unit 2 by the pump 31. During the circulation, the droplets are ejected into the poor solvent 62 from the ejection hole of the droplet-ejecting unit 2. By applying a flow to the poor solvent 62, cohesion of particles formed from the droplets 21 can be prevented.

Since the tank 63 includes the heating unit 33 and the decompression unit 36, moreover, the good solvent included in the poor solvent 62 can be removed. For example, the liquid is decompressed by the decompression unit 36 with heating the poor solvent using the heating unit 33. As a result, the good solvent, which has a boiling point lower than that of the poor solvent, is evaporated. The evaporated good solvent is condensed by a condenser 35 and is collected through a collecting tube 37. The heating by the heating unit 33 is performed not to change the bioactivity of the physiologically active substance included in the particles. Moreover, the heating unit may not be included.

[Second embodiment (gas drying)]

<Method for producing particles (second embodiment)>

A method for producing particles according to the second embodiment (gas drying) includes a droplet-ejecting step and a granulating step, and may further include other steps according to the necessity. The droplet-ejecting step includes ejecting droplets each including a physiologically active substance having bioactivity, a base material, and a solvent into gas. The granulating step includes evaporating the solvent included in the droplets to remove the solvent from the droplets to granulate particles.

Various methods have been known in the art as a dry granulation method where particles are granulated in gas, as in the second embodiment.

Examples thereof include an air pulverization method, such as a method where particle materials are homogeneously dispersed through melt-kneading, the resultant melt-kneaded product is cooled, and the melt-kneaded product is pulverized by a pulverizer to obtain pulverized particles having small particle diameters, and a method where a liquid including particle materials is freeze-dried, and the resultant is pulverized by a pulverizer to obtain pulverized particles having small particle diameters.

Moreover, the examples include a spray dry method, such as a method where a liquid including particle materials is sprayed in air to dry to obtain sprayed particles having small particle diameters. Examples of the spray method include a pressure nozzle method where the liquid is pressed to eject the liquid from a nozzle, and a disk method where the liquid is fed to a disk rotating at high speed to scatter the liquid by centrifugal force.

The air pulverization method is advantages because an equipment used for pulverization is simple, but it is difficult to produce having a narrow particle size distribution.

When the physiologically active substance whose bioactivity may be changed by heating is included as particle materials in the method for pulverizing the melt-kneaded product, moreover, the bioactivity of the physiologically active substance changes and, as a result, the level of the bioactivity reduces.

When the physiologically active substance whose bioactivity may be changed by cooling is included as particle materials in the method for pulverizing the freeze-dried product, moreover, the bioactivity of the physiologically active substance changes and, as a result, the level of the bioactivity reduces.

In the gas pulverization method, large external stress is generated during the pulverization process. In the case where the physiologically active substance whose bioactivity changes by application of the external stress is included as a material of the particles, therefore, bioactivity of the physiologically active substance changes, and as a result, the level of bioactivity reduces.

The spray drying method can produce particles, in which the physiologically active substance is held in each particles at a high proportion (physiologically active substance retention rate), but it is generally difficult to produce particles having small particle diameter.

When the method for spraying is disk spraying, particles having small particle diameters may be produced, but a large scale of equipment is used.

Moreover, the spray drying method cannot easily produce particles having a narrow particle size distribution because droplets tend to cohered to one another in gas. In order to prevent the droplets from being cohered to one another in the gas, heating is performed to quickly dry the droplets just after spraying. When the physiologically active substance whose bioactivity changes by heating is included as a material of the particles, the bioactivity of the physiologically active substance changes, and as a result, the level of the bioactivity reduced.

In the spray drying method, moreover, large external stress is generated by spraying to which high shear force is applied. In the case where the physiologically active substance whose bioactivity changes by application of the external stress is included as a material of the particles, therefore, bioactivity of the physiologically active substance changes, and as a result, the level of bioactivity reduces.

The method for producing particles of the second embodiment, which will be described hereinafter, is not an equivalent of the gas pulverization method or the spray drying method. Even in the case where a physiologically active substance whose bioactivity changes by heating, cooling, or application of external stress is included as a material of the particles, therefore, a change in the bioactivity of the physiologically active substance is prevented, and as a result, the level of bioactivity is not lowered.

The method for producing particles according to the second embodiment, which will be described hereinafter, is not an equivalent of the wet pulverization or two-liquid mixing. Even in the case where a physiologically active substance whose bioactivity changes by heating, cooling, or application of external stress is included as a material of the particles, therefore, a change in the bioactivity of the physiologically active substance is prevented, and as a result, the level of bioactivity is not lowered.

-Droplet-ejecting step (second embodiment)-

In the second embodiment, the droplet-ejecting step is a step including ejecting droplets each including a base material, a physiologically active substance having bioactivity, and solvent in gas. The droplet-ejecting step is identical to the droplet-ejecting step of the first embodiment, except that the solvent is used instead of the good solvent, and the droplets are ejected into the gas.

As an example of the droplet-ejecting step of the second embodiment, a method for ejecting a liquid including a base material, a physiologically active substance, and a solvent as droplets using vibrations will be described.

The method for ejecting the droplets using vibrations include the following methods. Each method will be described hereinafter.

(a) A method using a volume-changing unit configured to change a volume of a liquid storage unit using vibrations.
(b) A method using a constriction-generating unit configured to release a liquid from a plurality of ejection holes provided in the liquid storage unit with applying vibrations to the liquid storage unit, and to form the liquid into droplets from the column state to constriction state.
(c) A method using a nozzle-vibrating unit configured to a thin film in which vibrate nozzles are formed.

The volume-changing unit is not particularly limited and may be appropriately selected depending on the intended purpose, as long as the volume-changing unit can change a volume of the liquid storage unit. Examples thereof include a piezoelectric element that expands or contracts upon application of voltage (may be referred to as a "piezo element").

Examples of the constriction-generating unit include units using techniques disclosed in Japanese Unexamined Patent Application Publication No. 2007-199463. Japanese Unexamined Patent Application Publication No. 2007-199463 discloses a unit configured to release a liquid from a plurality of nozzle holes provided in a liquid storage unit with applying vibrations to the liquid storage unit using a piezoelectric element in contact with part of the liquid storage unit, to thereby turn the liquid into droplets from the column state to the constriction state.

Examples of the nozzle-vibrating unit include units using techniques disclosed in Japanese Unexamined Patent Application Publication No. 2008-292976. Japanese Unexamined Patent Application Publication No. 2008-292976 discloses a unit configured to release a liquid from a plurality of nozzle holes to form the liquid into droplets using a thin film disposed in a liquid storage unit and having a plurality of nozzles formed therein, and a piezoelectric element disposed in a region within which the thin film can be deformed and configured to vibrate the thin film.

As the unit for generating vibrations, a piezoelectric element is generally used. The piezoelectric element is not particularly limited, and a shape, size, and material thereof are appropriately selected. For example, a piezoelectric element used for a conventional inkjet ejection system can be suitably used.

The shape and size of the piezoelectric element are not particularly limited, and may be appropriately selected depending on a shape of the ejection hole.

A material of the piezoelectric element is not particularly limited and may be appropriately selected depending on the intended purpose. Examples of the material include: piezoelectric ceramics, such as lead zirconate titanate (PZT);

piezoelectric polymers, such as polyvinylidene fluoride (PVDF); and monocrystals, such as quartz, $LiNbO_3$, $LiTaO_3$, and $KNbO_3$.

The ejection hole is not particularly limited and may be appropriately selected depending on the intended purpose. Examples thereof include an opening provided in a nozzle plate.

A cross-sectional shape and size of the ejection hole are appropriately selected.

The cross-sectional shape of the ejection hole is not particularly limited and may be appropriately selected depending on the intended purpose. Examples thereof include: (1) such a tapered shape that an opening diameter is decreased from the inner part (the liquid storage unit side) of the ejection hole towards the outer part (the liquid ejection side); (2) such a shape that an opening diameter is narrowed with maintaining the round shape from the inner part (the liquid storage unit side) of the ejection hole towards the outer part (the liquid ejection side); (3) such a shape that an opening diameter is narrowed from the inner part (the liquid storage unit side) of the ejection hole towards the outer part (the liquid ejection side) with maintaining a constant nozzle angle; and (4) a combination of the shape of (1) and the shape of (2). Among the above-listed examples, the shape of (3) is preferable because the pressure applied to the liquid at the ejection hole becomes the maximum.

The nozzle angle of the shape of (3) is not particularly limited and may be appropriately selected depending on the intended purpose. The nozzle angle is preferably 60° or greater but 90° or less. When the nozzle angle is 60° or greater but 90° or less, ejection of droplets can be stabilized.

A size of the ejection hole is not particularly limited and may be appropriately selected depending on the intended purpose. For example, a diameter of the ejection hole is preferably smaller than 1,000 $\mu$m, more preferably 1.0 $\mu$m or greater but smaller than 1,000 $\mu$m, even more preferably 1.0 $\mu$m or greater but 500 $\mu$m or smaller, and particularly preferably 1.0 $\mu$m or greater but 50 $\mu$m or smaller. When the shape of the ejection hole is not a true circle, a diameter of a true circle having the same area to the area of the ejection hole is determined as the diameter of the ejection hole.

--Particle composition liquid--

The particle composition liquid includes the base material, the physiologically active substance having bioactivity, and a solvent. The same materials for the base material and physiologically active substance included in the particles are used for the base material and physiologically active substance included in the particle composition liquid. Therefore, description thereof is omitted, and only the solvent will be described.

---Solvent---

The solvent is a liquid in which the base material is dissolved. Moreover, the solvent is preferably a liquid that can also dissolve the physiologically active substance.

Examples of the solvent include water, aliphatic halogenated hydrocarbons (e.g., dichloromethane, dichloroethane, and chloroform), alcohols (e.g., methanol, ethanol, and propanol), ketones (e.g., acetone and methyl ethyl ketone), ethers (e.g., diethyl ether, dibutyl ether, and 1,4-dioxane), aliphatic hydrocarbons (e.g., n-hexane, cyclohexane, and n-heptane), aromatic hydrocarbons (e.g., benzene, toluene, and xylene), organic acids (e.g., acetic acid and propionic acid), esters (e.g., ethyl acetate), amides (e.g., dimethylformamide and dimethylacetamide), and mixed solvents of the above-listed solvents. The above-listed examples may be used alone or in combination. Among the above-listed examples, aliphatic halogenated hydrocarbons, alcohols, or mixed solvents thereof are preferable, and dichloromethane, 1,4-dioxane, methanol, ethanol, or mixed solvents thereof are more preferable in view of solubility.

An amount of the solvent is preferably 70% by mass or greater but 99.5% by mass or less, and more preferably 90% by mass or greater but 99% by mass or less, relative to a mass of the particle composition liquid. When the amount of the solvent is 70% by mass or greater but 99.5% by mass or less, production stability improves because of solubility and liquid viscosity of the particle materials.

The viscosity of the particle composition liquid is not particularly limited and may be appropriately selected depending on the intended purpose. The viscosity thereof is preferably 0.5 mPa•s or greater but 15.0 mPa•s or less, and more preferably 0.5 mPa•s or greater but 10.0 mPa•s or less. Note that, the viscosity can be measured, for example, by means of a viscoelasticity measurement device (device name: MCR rheometer, available from AntonPaar) at 25°C, and at a shear rate of 10 s$^{-1}$. The viscosity of the liquid being 0.5 mPa•s or greater but 15.0 mPa•s or less is preferable because droplets are desirably ejected from the above-described unit.

The surface tension of the particle composition liquid is not particularly limited and may be appropriately selected depending on the intended purpose. The surface tension thereof is preferably 10 mN/m or greater but 60 mN/m or less, and more preferably 20 mN/m or greater but 50 mN/m or less. Note that, the surface tension may be measured by a maximum foaming pressure method using, for example, a portable surface tensiometer (device name: POCKETDYNE, available from KRUSS) under the conditions of 25°C and a lifetime of 1,000 ms. The surface tension of the liquid being 0.5 mPa•s or greater but 15.0 mPa•s or less is preferable because droplets are desirably ejected from the above-described unit.

-Granulating step (second embodiment)-

In the second embodiment, the granulating step is a step including evaporating the solvent from the droplets to remove the solvent included in the droplets, to thereby granulate particles.

The granulating step is performed in gas. Specifically, the granulating step is preferably performed when the droplets ejected into the gas in the droplet-ejecting step travel through the gas.

Since the particles are granulated in the step as described above, the particles can be obtained as solid dispersions. Specifically, the particles, in each of which the physiologically active substance is dispersed in the base material, can be produced.

The particles produced by the method of the second embodiment do not need to be dried by heating or cooling, unlike a spray drying method known in the art. Therefore, the method of the second embodiment is advantageous particularly in formation of particles whose physiologically active substance easily changes by heating or cooling.

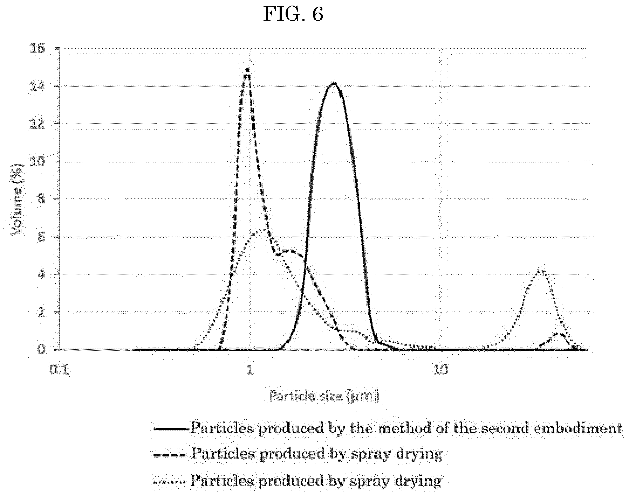

Since droplets having substantially the same size can be ejected with controlling not to cause cohesion of the droplets, and the solvent is evaporated from the droplets to granulate particles, a large amount of the particles having a uniformed size can be produced, and therefore a narrow particle size distribution of the particles can be obtained, as illustrated in FIG. 6.

FIG. 6 is a view illustrating an example of a particle size distribution of the particles produced by the method of the second embodiment, and an example of a particle size distribution of the particles produced by a spray drying method. As depicted in FIG. 6, the particles produced by the method of the second embodiment has a particle size distribution having only one narrow peak without a peak indicating the presence of coarse particles, unlike particles produced by a spray drying method.

Moreover, the particle diameters of the particles can be adjusted by appropriately adjusting a size of the ejection hole of the ejection unit configured to form droplets.

As a member for reducing a particle size of particles, an ejection unit configured to form droplets by vibrations etc. is used instead of a pulverization device that generates large external stress, or a spraying device that applies high shear force. Even in the case where a physiologically active substance whose bioactivity changes by application of external stress is included as a material of the particles, therefore, a change in the bioactivity of the physiologically active substance is prevented, and as a result, the level of bioactivity is not lowered.

In the method of the second embodiment, moreover, particles are not in contact with a solvent, such as water, at the time of granulation. Therefore, particles produced has a high proportion of the physiologically active substance (retention of physiologically active substance) held inside each particle after the production of the particles.

Compared with other methods, the method of the second embodiment can improve a bioactivity rate of the particles. For example, the bioactivity rate can be improved to 50% or greater.

In the granulating step, particles may be granulated by ejecting droplets into a transport gas flow to evaporate the solvent from the droplets. A method for evaporating the solvent from the droplets using the transport gas flow is not particularly limited and may be appropriately selected depending on the intended purpose. As the method, for example, preferred is a method for arranging a traveling direction of the transport gas flow to be substantially vertical to the direction along which droplets are ejected.

A temperature, vapor pressure, and gas of the transport gas flow are preferably appropriately adjusted. A heating unit may be disposed in order to adjust a temperature of the transport gas flow. As described above, ejection of the droplets is performed in the granulating step in a manner that cohesion of the droplets is suppressed. Therefore, the degree of heat applied by the heating unit can be reduced. Specifically, heating can be performed in a manner that the bioactivity of the physiologically active substance does not change by the heating.

Moreover, the solvent may not be completely evaporated, as long as the collected particles can maintain a solid state. A drying step may be added as a separate step after the collection of the particles. Moreover, a method for evaporating the solvent from the droplets using a temperature change or chemical change may be used.

-Other steps-

Other steps are not particularly limited and may be appropriately selected depending on the intended purpose. Examples thereof include a particle collecting step.

The particle collecting step is a step including collecting the produced particles. The particle collecting step is preferably performed by a particle collecting unit. The particle collecting unit is not particularly limited and may be appropriately selected depending on the intended purpose. Examples thereof include a cyclone collector, and a back filter.

When particles each including at least two base materials, where one of the two or more base materials is locally arranged at the surface side of each particle, are produced, such particles can be formed in the granulating step by appropriately selecting base materials to be included in the particle composition liquid.

When particles, where one of the two or more base materials is locally arranged at the surface side of each particle, are produced in the granulating step, the base materials for use have mutually different contact angles. As a result, interaction between the base materials increases as the solvent is evaporated from the droplets in the granulating step.

Since the base materials for use have mutually different contact angles, phase separation between the base materials easily occurs. As a result, one of the at least two base materials is locally included at the surface side of each droplet. As the evaporation of the solvent progresses, the droplet is solidified in the above-mentioned state to thereby form a particle. According to the method as described, particles, where one of the two or more base materials is locally arranged at the surface side of each particle, can be formed by only one step.

The difference in the contact angle between the base materials is not particularly limited and may be appropriately selected depending on the intended purpose. The difference is preferably 1.0° or greater, and more preferably 10.0° or greater. When the difference in the contact angle between the base materials is within the above-mentioned preferable range, phase separation between the base materials easily occurs.

A method for measuring the contact angle of the base material is not particularly limited and may be appropriately selected depending on the intended purpose. Examples thereof include a measurement method using a contact angle meter. Examples of the contact angle meter include a pocket goniometer PG-X+/mobile contact angle analyzer, available from FIBRO system.

A method for confirming the presence of the phase separation between the at least two base materials is not particularly limited and may be appropriately selected depending on the intended purpose. Examples thereof include a method where a solution, in which at least two base materials are dissolved in a good solvent, is applied into the form of a thin film by a bar coater, and the state of the film is confirmed under an optical microscope before, during, and after drying. Examples of the optical microscope include OLYMPUS BX51 available from Olympus Corporation.

When the solvent used with the base materials is lipophilic, one base material locally arranged at the surface side of each particle has a contact angle larger than a contact angle of another base material locally arranged a the inner side of each particle. Since the one base material having the large contact angle has greater affinity to the solvent than another base material locally arranged at the inner side of each particle, the one base material having the large contact angle tends to be included at the side close to the solvent, i.e., the surface side of each particle.

When the solvent used with the base materials is hydrophilic, one base material locally arranged at the surface side of each particle has a contact angle smaller than a contact angle of another base material locally arranged a the inner side of each particle. Since the one base material having the small contact angle has less affinity to the solvent than another base material locally arranged at the inner side of each particle, the one base material having the large contact angle tends to be included at the side close to the solvent, i.e., the surface side of each particle.

When particles including a water-soluble physiologically active substance are produced, such as in case of most of pharmaceutical compositions, for example, a structure where the physiologically active substance is included at the inner side of each particle, and the inner side of each particle is coated with one base material locally arranged at the surface side of each particle can be obtained by using a lipophilic solvent.

When particles including an oil-soluble physiologically active substance are produced, such as in case of most of pharmaceutical compositions, for example, a structure where the physiologically active substance is included at the inner side of each particle, and the inner side of each particle is coated with one base material locally arranged at the surface side of each particle can be obtained by using a hydrophilic solvent.

Examples of the one base material locally included at the surface side of each particle include a pH-dependent-release material. Since the pH-dependent-release material is used, for example, particles that can be added to an enteric pharmaceutical composition can be produced.

Examples of the pH-dependent-release material include a cellulose-based polymer, and a methacrylic acid-based polymer. Since the cellulose-based polymer and the methacrylic acid-based polymer have larger contact angles than contact angles of other base materials, particles having a structure where the physiologically active substance is included at the inner side of each particle, and the inner side of each particle is coated with one base material locally arranged at the surface side of each particle can be formed. The above-listed examples may be used alone or in combination.

Among the cellulose-based polymers, hydroxypropyl methylcellulose acetate succinate, and hydroxypropyl methylcellulose phthalate are preferable. Since the above-listed cellulose-based polymers have larger contact angles than contact angles of other base materials, particles having a structure where the physiologically active substance is included at the inner side of each particle, and the inner side of each particle is coated with one base material locally arranged at the surface side of each particle can be formed. The above-listed examples may be used alone or in combination.

Among the methacrylic acid-based polymers, moreover, an ammonioalkyl methacrylic acid ester copolymer is preferable because the ammonioalkyl methacrylic acid ester copolymer has a contact angle larger than contact angles of other base materials.

Examples of a combination of the one base material locally arranged at the surface side of each particle and another base material locally arranged at the inner side of each particle include a combination of (i) at least one selected from the group consisting of poly(meth)acrylic acid, polyglycolic acid, and hydroxypropyl methyl cellulose, and (ii) at least one selected from the group consisting of hydroxypropyl cellulose, polyethylene pyrrolidone, and polyalkylene glycol. Such a combination of the base materials are not compatible to each other and causes phase separation.

In addition to those mentioned above, examples of the above-mentioned other steps include a step including, after granulation of the particles, passing the particles through a filter or a sieve to obtain particles having a uniform size.

<Apparatus for producing particles (second embodiment)>

In the second embodiment (gas drying), the apparatus for producing particles include a droplet-ejecting unit and a granulating unit, and may further include other units according to the necessity. The droplet-ejecting unit is configured to eject droplets each including a physiologically active substance having bioactivity, a base material, and a solvent, into gas. The granulating unit is configured to evaporate the solvent from the droplets to remove the solvent from the droplets to thereby granulate particles.

-Droplet-ejecting unit (second embodiment)-

The droplet-ejecting unit is a unit configured to eject a liquid including a physiologically active substance, a base material, and a solvent into gas to form droplets.

The droplet-ejecting unit is as described in the description of the droplet-ejecting unit used in the apparatus for producing particles of the first embodiment. As a preferable embodiment, the droplet-ejecting unit is configured to apply vibration to eject the particle composition liquid to form droplets.

The droplet-ejecting unit is connected to a liquid storage container, which will be described later. A member for connecting between the droplet-ejecting unit and the liquid storage container is not particularly limited and may be appropriately selected depending on the intended purpose, as long as the liquid can be supplied from the liquid storage container to the droplet-ejecting unit. Examples of such member include a pipe, and a tube.

The droplet-ejecting unit preferably includes a vibration applying member configured to apply vibrations to the liquid to eject the liquid in the form of droplets. The vibrations are not particularly limited and may be appropriately selected depending on the intended purpose. For example, the frequency is preferably 1 kHz or greater, more preferably 150 kHz or greater, and even more preferably 300 kHz or greater but 500 kHz or less. When the vibrations are 1 kHz or greater, liquid columns ejected from the ejection holes can be formed into droplets with good reproducibility. When the vibrations are 150 kHz or greater, production efficiency can be improved.

Examples of the droplet-ejecting unit including the vibration applying member include inkjet nozzles. As the ejection mechanism of the inkjet nozzles, for example, a liquid column resonance method, a membrane vibration method, a liquid vibration method, a Rayleigh breakup method, etc. may be used.

--Liquid storage container--

The liquid storage container is a container, in which the liquid including the base material, the physiologically active substance, and the good solvent is stored.

The liquid storage container may be or may not be flexible. A material of the liquid storage container is not particularly limited and may be appropriately selected depending on the intended purpose. The material thereof may be a resin or a metal. A structure of the liquid storage container is not particularly limited and may be appropriately selected depending on the intended purpose. Examples of the structure thereof include a sealed structure or a non-sealed structure.

-Granulating unit (second embodiment)-

The granulating unit is a unit configured to evaporate the solvent from the droplets to remove the solvent included in the droplets, to thereby granulate particles.

Examples of the granulating unit include a member configured to form a space for evaporating the solvent from the droplets.

The granulating unit preferably includes a transport gas flow forming unit configured to form transport gas flows.

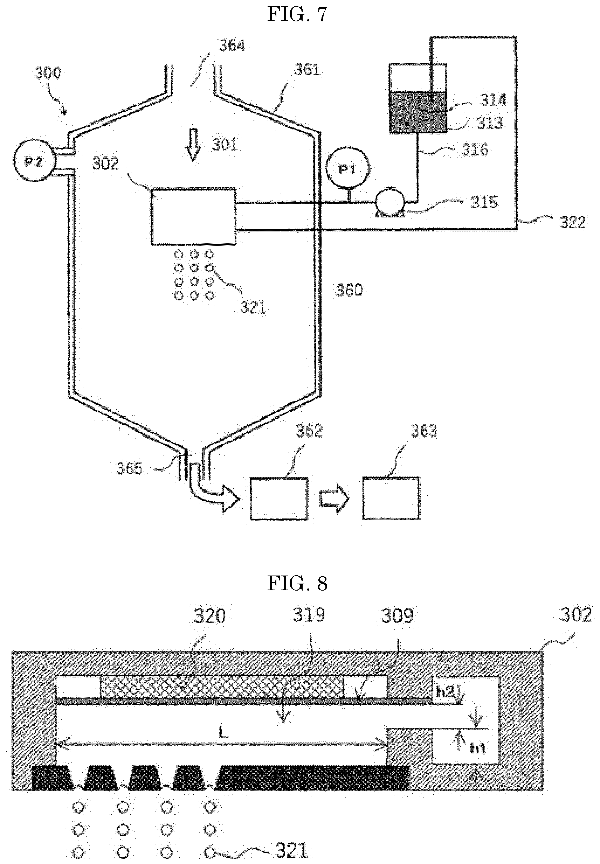

Next, specific examples of the second embodiment will be described with reference to FIGs. 7 to 9.

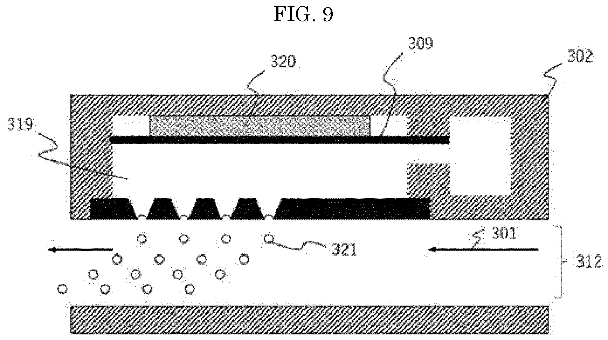

FIG. 7 is a schematic view illustrating one example of the apparatus for producing particles. FIG. 8 is a schematic cross-sectional view illustrating one example of a droplet-ejecting unit used in the apparatus for producing particles. FIG. 9 is a schematic cross-sectional view illustrating one example of the droplet-ejecting unit used in the apparatus for producing particles.

The apparatus 300 for producing particles of FIG. 7 includes a droplet-ejecting unit 302, a dry collecting unit 360, a transport gas flow outlet 365, and a particle storage unit 363. To the droplet-ejecting unit 302, a liquid storage container 313, in which a liquid 314 is stored, and a liquid circulation pump 315 are connected. The liquid-circulating pump 315 is configured to supply the liquid 314 stored in the liquid storage container 313 to the droplet-ejecting unit 302 via the liquid supply tube 316. Moreover, the liquid-circulating pump 315 is configured to pressure feed the liquid inside the liquid supply tube 316 to return to the liquid storage container 313 via a liquid returning tube 322. Therefore, the liquid 314 can be supplied to the droplet-ejecting unit 302 as needed. A pressure gauge P1 is disposed to the liquid supply tube 316 and a pressure gauge P2 is disposed to the dry collecting unit. The feeding pressure to the droplet-ejecting unit 302 is managed by the pressure gauge P1, and the internal pressure of the dry collecting unit is managed by the pressure gauge P2. When the measured pressure value of P1 is larger than the measured pressure value of P2, the liquid 314 may be bled out from the ejection hole. When the measured pressure value of P1 is smaller than the measured pressure value of P2, gas may enter the droplet-ejecting unit 302 to stop ejection of the liquid. Therefore, the measured pressure value of P1 and the measured pressure value of P2 are preferably substantially the same.

A descending gas stream (transport gas flow) 301 from the transport gas flow inlet 364 is formed within a chamber 361. The droplets 321 ejected from the droplet-ejecting unit 302 is transported downwards not only by gravity but also the transport gas flow 301, passed through a transport gas flow outlet 365, collected by a particle-collecting unit 362, and stored in a particle storage unit 363.

When ejected droplets are brought into contact with each other before drying in the droplet-ejecting step, cohesion of the droplets may occur. In order to obtain particles having a narrow particle size distribution, it is desirable to keep the ejected droplets apart from one another. The ejected droplets travel at a certain initial speed, but the traveling speed eventually slows down due to air resistance. When the subsequent droplets catch up with the preceding droplets, which have slow down and are not sufficiently dried, cohesion of the droplets occurs. In order to prevent cohesion of the droplets, the droplets are preferably dried and transported by the transport gas flow 301 in a manner that the droplets are prevented from being in contact with one another to prevent cohesion of the droplets. To this end, the transport gas flow 301 is preferably arranged along the same direction to the ejection direction of the droplets adjacent to the droplet-ejecting unit 302. Even when the droplets are brought into contact with one another, cohesion does not occur if the droplets are completely dried prior to the contact. In such a case, the transport gas flow 301 may not be used.

FIG. 8 is an enlarged view of the droplet-ejecting unit of the apparatus for producing particles of FIG. 7. As illustrated in FIG. 8, the droplet-ejecting unit 302 includes a volume-changing unit 320, an elastic plate 309, and a liquid storage unit 319. Since the droplet-ejecting unit 302 is deformed to reduce a volume of the liquid storage unit 319 as voltage is applied to the volume-changing unit 320, the liquid stored in the liquid storage unit 319 is ejected as droplets 321 from the ejection holes.

FIG. 9 is a view illustrating another embodiment of the droplet-ejecting unit of the apparatus for producing particles. As illustrated in FIG. 9, the direction of the transport gas flow 301 in the gas flow path 312 may be substantially vertical to the ejection direction. The transport gas flow 301 may be set to have an angle. The transport gas flow 301 is preferably set to have an angle with which droplets travel away from the droplet-ejecting unit 302. When the droplets 321 are ejected by changing the volume of the liquid storage unit 319 by the volume-changing unit 320 via the elastic plate 309 and a transport gas flow 301 for preventing cohesion is introduced to the ejected droplets 321 from the direction substantially vertical to the direction of the ejection of the droplets, as illustrated in FIG. 9, the ejection holes are preferably arranged not to overlap trajectories of the droplets when the droplets 321 are transported from the ejection holes by the transport gas flow 301 for preventing cohesion.

After preventing cohesion of the droplets by the transport gas flow 301, the resultant particles may be transported to the particle-collecting unit by another gas flow.

The speed of the transport gas flow is preferably the same as or faster than the ejection speed of the droplets. When the speed of the transport gas flow is faster than the ejection speed of the droplet, cohesion between the droplets can be prevented.

Moreover, a chemical substance for accelerating the process for drying the droplets may be added to the transport gas flow. The state of the transport gas flow is not limited. The transport gas flow may be a laminar flow, a swirl flow, or a turbulent flow. A type of the gas constituting the transport gas flow is not particularly limited and may be appropriately selected depending on the intended purpose. As the gas constituting the transport gas flow, air may be used, or incombustible gas, such as nitrogen, may be used.

Moreover, a temperature of the transport gas flow can be appropriately adjusted. The temperature thereof is a temperature at which the bioactivity of the physiologically active substance included in the droplets does not change.

When an amount of the residual solvent in the particles collected by the particle-collecting unit 362 of FIG. 7 is large, secondary drying is preferably performed, if necessary, in order to reduce the amount of the residual solvent. For the secondary drying, a typical drying method known in the art, such as fluidized bed drying and vacuum drying, can be used.

Examples

The present disclosure will be described more detail by way of Examples. However, the present disclosure should not be construed as being limited to these Examples.

(Example 1)

-Preparation of Liquid Formulation A-

To dichloromethane serving as a solvent, ibuprofen (available from Tokyo Chemical Industry Co., Ltd.) serving as a physiologically active substance and polylactic acid glycolic acid (PLGA5020, available from FUJIFILM Wako Pure Chemical Corporation) serving as a base material were added, to thereby prepare Liquid Formulation A. Liquid Formulation A was prepared in a manner that an amount of the ibuprofen was 0.03% by mass, and an amount of the polylactic acid glycolic acid was 0.27% by mass relative to a total amount of Liquid Formulation A.

-Granulation of Particles 1 (gas drying)-

Liquid Formulation A was ejected from an ejection hole to form droplets using the Rayleigh breakup droplet-ejecting unit disclosed in Japanese Patent No. 4647506, and the solvent was removed from the droplets using the apparatus for producing particles illustrated in FIG. 7, to thereby granulate Particles 1.
Particles 1 obtained had a ratio (surface area/volume) of 0.25, the number average particle diameter (Dn) of 18.2 $\mu$m, and R.S.F. of 0.9. The values as mentioned were measured by means of a laser diffraction/scattering particle size distribution analyzer (device name: LA-960, available from HORIBA, Ltd.). The ratio (surface area/volume), the number average particle diameter, and the R.S.F. were measured in the same manner hereinafter. The production conditions of the particles were as follows.

[Production conditions of particles]

Shape of ejection hole: true circle
Diameter of ejection hole: 30 $\mu$m
Extrusion pressure of liquid formulation: 0.18 MPa
Excitation frequency: 70 kHz
Excitation voltage: 5 V
Transport gas flow rate: 50 m$^3$/h

(Example 2)

-Granulation of Particles 2 (spray drying)-

Particles 2 were granulated in the same manner as in Example 1, except that Liquid Formulation A obtained was ejected using a spray drying unit (2-fluid nozzle, available from Yamato Scientific Co., Ltd.) under the following production conditions of particles.
Particles 2 obtained had the ratio of 0.60, the number average particle diameter (Dn) of 12.1 $\mu$m, and R.S.F. of 1.0. The production conditions of the particles were as follows.

[Production conditions of particles]

Feeding rate of Liquid Formulation A to nozzle: 10 mL/min
Dry gas flow rate (dry nitrogen): 30 L/min
Orifice pressure: 1.3 kPa
Temperature (Inlet): 50°C
Temperature (Outlet): 40°C

(Example 3)

-Granulation of Particles 3 (Rayleigh breakup)-

Particles 3 were granulated in the same manner as in Example 1, except that Liquid Formulation A obtained was granulated under the following production conditions of particles.

Particles 3 obtained had the ratio of 0.54, the number average particle diameter (Dn) of 10.0 $\mu$m, and R.S.F. of 0.8. The production conditions of the particles were as follows.

[Production conditions of particles]

Shape of ejection hole: true circle
Diameter of ejection hole: 20 $\mu$m
Extrusion pressure of liquid formulation: 0.18 MPa
Excitation frequency: 70 kHz
Excitation voltage: 5 V
Transport gas flow rate: 50 m$^3$/h

(Example 4)

-Granulation of Particles 4 (Rayleigh breakup)-

Particles 4 were granulated in the same manner as in Example 1, except that Liquid Formulation A obtained was granulated under the following production conditions of particles.

Particles 3 obtained had the ratio of 0.21, the number average particle diameter (Dn) of 30.0 $\mu$m, and R.S.F. of 0.7. The production conditions of the particles were as follows.

[Production conditions of particles]

Shape of ejection hole: true circle
Diameter of ejection hole: 45 $\mu$m
Extrusion pressure of liquid formulation: 0.18 MPa
Excitation frequency: 70 kHz
Excitation voltage: 5 V
Transport gas flow rate: 50 m$^3$/h

(Example 5)

-Granulation of Particles 5 (spray drying)-

Particles 5 were granulated in the same manner as in Example 1, except that Liquid Formulation A obtained was ejected using a spray drying unit (2-fluid nozzle, available from Yamato Scientific Co., Ltd.) under the following production conditions of particles.

Particles 5 obtained had the ratio of 0.58, the number average particle diameter (Dn) of 11.5 $\mu$m, and R.S.F. of 1.1. The production conditions of the particles were as follows.

[Production conditions of particles]

Feeding rate of Liquid Formulation A to nozzle: 10 mL/min
Dry gas flow rate (dry nitrogen): 30 L/min
Orifice pressure: 1.3 kPa
Temperature (Inlet): 50°C
Temperature (Outlet): 40°C

(Example 6)

-Granulation of Particles 6 (Rayleigh breakup)-

Particles 6 were granulated in the same manner as in Example 1, except that the polylactic acid glycolic acid was replaced with an ammonio alkyl methacrylate copolymer (EUDRAGIT RSPO, available from Evonik) to prepare Liquid Formulation B, and Liquid Formulation B obtained was used for the granulation.

Particles 6 obtained had the ratio of 0.28, the number average particle diameter (Dn) of 19.4 $\mu$m, and R.S.F. of 0.8. The production conditions of the particles were as follows.

[Production conditions of particles]

Shape of ejection hole: true circle
Diameter of ejection hole: 30 $\mu$m
Extrusion pressure of liquid formulation: 0.18 MPa
Excitation frequency: 70 kHz
Excitation voltage: 5 V
Transport gas flow rate: 50 m$^3$/h

(Example 7)

-Granulation of Particles 7 (Rayleigh breakup)-

Particles 7 were granulated in the same manner as in Example 1, except that the ibuprofen was replaced with cyclosporine (Cyclosporin A, available from Wako Pure Chemical Industries, Ltd.) to prepare Liquid Formulation C, and Liquid Formulation C obtained was used for the granulation.
Particles 7 obtained had the ratio of 0.27, the number average particle diameter (Dn) of 18.9 $\mu$m, and R.S.F. of 0.9. The production conditions of the particles were as follows.

[Production conditions of particles]

Shape of ejection hole: true circle
Diameter of ejection hole: 30 $\mu$m
Extrusion pressure of liquid formulation: 0.18 MPa
Excitation frequency: 70 kHz
Excitation voltage: 5 V
Transport gas flow rate: 50 m$^3$/h

(Comparative Example 1)

-Granulation of Particles 8 (spray drying)-

Particles 8 were granulated in the same manner as in Example 1, except that Liquid Formulation A obtained was ejected using a spray drying unit (4-fluid nozzle, available from Fujisaki Electric Co., Ltd.).
Particles 8 obtained had the ratio of 0.62, the number average particle diameter (Dn) of 10.4 $\mu$m, and R.S.F. of 1.0. The production conditions of the particles were as follows.

[Production conditions of particles]

Feeding rate of Liquid Formulation A to nozzle: 15 mL/min
Dry gas flow rate (dry nitrogen): 30 L/min
Orifice pressure: 1.3 kPa
Temperature (Inlet): 50°C
Temperature (Outlet): 40°C

(Comparative Example 2)

-Granulation of Particles 9 (spray drying)-

Particles 9 were granulated in the same manner as in Example 1, except that Liquid Formulation A obtained was ejected using a spray drying unit (4-fluid nozzle, available from Fujisaki Electric Co., Ltd.).
Particles 9 obtained had the ratio of 0.73, the number average particle diameter (Dn) of 6.7 $\mu$m, and R.S.F. of 1.0. The production conditions of the particles were as follows.

[Production conditions of particles]

Feeding rate of Liquid Formulation A to nozzle: 10 mL/min
Dry gas flow rate (dry nitrogen): 30 L/min

Orifice pressure: 1.3 kPa
Temperature (Inlet): 50°C
Temperature (Outlet): 40°C

(Comparative Example 3)

-Granulation of Particles 10 (spray drying)-

Particles 10 were granulated in the same manner as in Example 1, except that Liquid Formulation A obtained was ejected using a spray drying unit (4-fluid nozzle, available from Fujisaki Electric Co., Ltd.).
Particles 10 obtained had the ratio of 1.58, the number average particle diameter (Dn) of 2.9 $\mu$m, and R.S.F. of 0.9. The production conditions of the particles were as follows.

[Production conditions of particles]

Feeding rate of Liquid Formulation A to nozzle: 7 mL/min
Dry gas flow rate (dry nitrogen): 30 L/min
Orifice pressure: 1.3 kPa
Temperature (Inlet): 50°C
Temperature (Outlet): 40°C
Next, "inclusion rate (%) of physiologically active substance" and "dissolution rate (%) of physiologically active substance" were measured and evaluated on Particles 1 to 10 obtained in Examples 1 to 7 and Comparative Examples 1 to 3. The results are presented in Table 1-3.

<Measurement of dissolution rate (%) of physiologically active substance>

A dissolution rate (%) of the physiologically active substance was measured in the following manner. A 50mL conical tube was charged with 20 mg of the particles and 30 mL of water, and was shaken using a shaker (digital shaker, available from FRONT LAB) at 100 rpm. The liquid inside the conical tube was collected after 1 hour, after 3 hours, and after 24 hours each by 1 mL. After performing centrifugal separation on the collected liquid, 0.5 mL of the supernatant was collected in a 2 mL vial. To the 2 mL vial, 0.5 mL of an acetonitrile aqueous solution (acetonitrile:water = 9:1) was added.
An amount of the eluted physiologically active substance was measuring using the above-prepared solution by liquid chromatography under the following conditions. A "dissolution rate (%)" was calculated from the measured elution amount based on the following equation. The results are presented in Table 1-3.

[Conditions of liquid chromatography]

Column: XBridge C18 3.5 $\mu$m (Waters) (Particle size: 3.5 $\mu$m, column size: 4.6 mm $\times$ 150 mm)
Column temperature: 80°C
Mobile phase: (A) pure water, (B) acetonitrile, A:B = 10:90
Injection amount: 10 pL
Detector: UV-VIS detector (Agilent Technologies); 210 nm
Flow rate: 1.0 mL/min

[Calculation equation for dissolution rate (%)]

$$\text{Dissolution rate } (\%) = (\text{amount of physiologically active substance in test liquid of elution test/amount of physiologically active substance present in granulated particles}) \times 100(\%)$$

<Measurement of inclusion rate (%) of physiologically active substance>

In order to measure a proportion of the physiologically active substance in the granulated particles, the particles were weight and collected by 2 mg. The collected particles were dissolved in 10 mL of the above-mentioned acetonitrile aqueous solution (acetonirtile:water = 9:1). A quantitative evaluation of the physiologically active substance was performed by liquid chromatography using the solution. The inclusion rate (%) was calculated using the following equation. The conditions of the liquid chromatography were the same as the conditions for the measurement of the dissolution rate (%).

[Calculation equation of inclusion rate (%)]

$$\text{Inclusion rate (\%)} = (\text{amount of physiologically active substance in granulated}$$
$$\text{particles/charged amount of physiologically active substance}) \times 100(\%)$$

Table 1-1

| | | | Liquid Formulation | | | |
|---|---|---|---|---|---|---|
| | | | Physiologically active substance | | Base material | | Solvent |
| | | | Type | Amount (mass%) | Type | Amount (mass%) | Type |
| Example | 1 | ibuprofen | 0.03 | PLGA | 0.27 | dichloromethane |
| | 2 | ibuprofen | 0.03 | PLGA | 0.27 | dichloromethane |
| | 3 | ibuprofen | 0.03 | PLGA | 0.27 | dichloromethane |
| | 4 | ibuprofen | 0.03 | PLGA | 0.27 | dichloromethane |
| | 5 | ibuprofen | 0.03 | PLGA | 0.27 | dichloromethane |
| | 6 | ibuprofen | 0.03 | Eudragit-RLPO | 0.27 | dichloromethane |
| | 7 | cyclosporin | 0.03 | PLGA | 0.27 | dichloromethane |
| Comparative Example | 1 | ibuprofen | 0.03 | PLGA | 0.27 | dichloromethane |
| | 2 | ibuprofen | 0.03 | PLGA | 0.27 | dichloromethane |
| | 3 | ibuprofen | 0.03 | PLGA | 0.27 | dichloromethane |

Table 1-2

| | | Method for producing particles | Particles | | |
|---|---|---|---|---|---|
| | | | Surface area/ volume | Number average particle diameter [Dn] ($\mu$m) | R.S.F |
| Example | 1 | Rayleigh breakup | 0.25 | 18.2 | 0.9 |
| | 2 | spray drying | 0.6 | 12.1 | 1.0 |
| | 3 | Rayleigh breakup | 0.54 | 10 | 0.8 |
| | 4 | Rayleigh breakup | 0.21 | 30 | 0.7 |
| | 5 | spray drying | 0.58 | 11.5 | 1.1 |
| | 6 | Rayleigh breakup | 0.28 | 19.4 | 0.8 |
| | 7 | Rayleigh breakup | 0.27 | 18.9 | 0.9 |

(continued)

| | | Method for producing particles | Particles | | |
|---|---|---|---|---|---|
| | | | Surface area/ volume | Number average particle diameter [Dn] ($\mu$m) | R.S.F |
| Comparative Example | 1 | spray drying | 0.62 | 10.4 | 1.0 |
| | 2 | spray drying | 0.73 | 6.7 | 1.0 |
| | 3 | spray drying | 1.58 | 2.9 | 0.9 |

Table 1-3

| | | Evaluation results | | | |
|---|---|---|---|---|---|
| | | Inclusion rate (%) | Dissolution rate after 1h. (%) | Dissolution rate after 3h. (%) | Dissolution rate after 24h. (%) |
| Example | 1 | 98 | 1.5 | 2.7 | 14.6 |
| | 2 | 97 | 5.2 | 8.4 | 18.5 |
| | 3 | 98 | 4.9 | 10.3 | 17.4 |
| | 4 | 97 | 1.1 | 2.1 | 10.7 |
| | 5 | 99 | 6.8 | 10.2 | 19.4 |
| | 6 | 96 | 9.6 | 17.4 | 47.2 |
| | 7 | 96 | 2.2 | 3.8 | 9.5 |
| Comparative Example | 1 | 96 | 6.2 | 10.1 | 20.9 |
| | 2 | 97 | 6.7 | 15.9 | 32.4 |
| | 3 | 95 | 24.9 | 30.5 | 41.6 |

As presented in Tables 1-1 to 1-3, it was found from the measurement results of the dissolution rate (%) that the dissolution rates of Examples having the small values in the ratio (surface area/volume) were lower than the dissolution rates of Comparative Examples at any timing. Accordingly, it was understood that the particles of Examples could prevent initial burst, and were particles suitable for a sustained-release pharmaceutical preparation.

For example, embodiments of the present disclosure are as follows.

<1> Sustained-release particles, each including:

a base material; and
a physiologically active substance,
wherein the ratio of the surface area of the sustained-release particles to the volume of the sustained-release particles is 0.6 or less.

<2> The sustained-release particles according to <1>,
wherein the number average particle diameter of the sustained-release particles is 1 $\mu$m or greater but 50 $\mu$m or less, and
a relative span factor (R.S.F) of the sustained-release particles is 1.0 or less.
<3> The sustained-release particles according to <1> or <2>,
wherein the base material includes a biodegradable resin.
<4> The sustained-release particles according to <3>,
wherein the biodegradable resin includes polylactic acid, or a polylactic acid-glycolic acid copolymer, or both.
<5> A method for producing sustained-release particles, the method including:

ejecting droplets each including a physiologically active substrate, a base material, and a solvent; and
removing the solvent from the droplets to granulate sustained-release particles,

wherein the sustained-release particles are the sustained-release particles according to any one of <1> to <4>, and the physiologically active substrate and the base material are the physiologically active substrate and the base material of the sustained-release particles according to any one of <1> to <4>.

<6> The method according to <5>, further including, after the removing, classifying the sustained-release particles into groups according to a size of the sustained-release particles.

The sustained-release particles according to any one of <1> to <4>, and the method for producing sustained-release particles according to <5> or <6> can solve the above-described various problems existing in the art and can achieve the object of the present disclosure.

**Claims**

1. Sustained-release particles, each comprising:

   a base material; and
   a physiologically active substance,
   wherein the ratio of the surface area of the sustained-release particles to the volume of the sustained-release particles is 0.6 or less.

2. The sustained-release particles according to claim 1,
   wherein the number average particle diameter of the sustained-release particles is 1 $\mu$m or greater but 50 $\mu$m or less, and
   a relative span factor R.S.F of the sustained-release particles is 1.0 or less.

3. The sustained-release particles according to claim 1 or 2,
   wherein the base material includes a biodegradable resin.

4. The sustained-release particles according to claim 3,
   wherein the biodegradable resin includes polylactic acid, or a polylactic acid-glycolic acid copolymer, or both.

5. A method for producing sustained-release particles, the method comprising:

   ejecting droplets each including a physiologically active substrate, a base material,
   and a solvent; and
   removing the solvent from the droplets to granulate sustained-release particles,
   wherein the sustained-release particles are the sustained-release particles according to any one of claims 1 to 4, and the physiologically active substrate and the base material are the physiologically active substrate and the base material of the sustained-release particles according to any one of claims 1 to 4.

6. The method according to claim 5, further comprising, after the removing, classifying the sustained-release particles into groups according to a size of the sustained-release particles.

FIG. 1

FIG. 2

FIG. 3

FIG. 4A

FIG. 4B

FIG. 5

FIG. 6

Particle size (μm)

——— Particles produced by the method of the second embodiment
- - - - Particles produced by spray drying
········· Particles produced by spray drying

FIG. 7

FIG. 8

FIG. 9

| Europäisches Patentamt European Patent Office Office européen des brevets | **EUROPEAN SEARCH REPORT** | Application Number EP 21 16 2145 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/150692 A1 (RICOH CO LTD [JP]; SHIZUOKA PREFECTURAL UNIV CORPORATION [JP] ET AL.) 8 September 2017 (2017-09-08) * paragraph [0008] * * paragraph [0011] * * page 33 - page 35; examples 1-5 * * claims * * figures * | 1-6 | INV. A61K9/16 |
| X | WO 2020/027030 A1 (RICOH CO LTD [JP]; SHIZUOKA PREFECTURAL UNIV CORPORATION [JP] ET AL.) 6 February 2020 (2020-02-06) * paragraph [0101] - paragraph [0108] * * page 39 - page 45; examples 1-6 * * page 47, paragraph 0176; table 2 * * claims * * figures * | 1-6 | |
| X | US 2018/085314 A1 (MORINAGA TADAHIKO [JP] ET AL) 29 March 2018 (2018-03-29) * paragraph [0007] - paragraph [0009] * * page 9 - page 10; example 1 * * page 10; example 5 * * page 11; table 1 * * claims * * figures * | 1-6 | TECHNICAL FIELDS SEARCHED (IPC) A61K |

-/--

The present search report has been drawn up for all claims

| Place of search | Date of completion of the search | Examiner |
|---|---|---|
| The Hague | 30 July 2021 | Muller, Sophie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 16 2145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIROKI SUZUKI ET AL: "Amorphous solid dispersion of cyclosporine A prepared with fine droplet drying process: Physicochemical and pharmacokinetic characterization", INTERNATIONAL JOURNAL OF PHARMACEUTICS, vol. 519, no. 1-2, 16 January 2017 (2017-01-16), pages 213-219, XP55601207, NL ISSN: 0378-5173, DOI: 10.1016/j.ijpharm.2017.01.018 * abstract * * 2.2. ASD formulation of CsA; page 214 * * 3.1. Appearance and particle size distribution; page 215 - page 216 * * page 216; figure 3 * * 3.3 Dissolution property; page 217 * ----- | 1-6 | |

| | | | TECHNICAL FIELDS SEARCHED (IPC) |

The present search report has been drawn up for all claims

| Place of search | Date of completion of the search | Examiner |
|---|---|---|
| The Hague | 30 July 2021 | Muller, Sophie |

EPO FORM 1503 03.82 (P04C01)

## ANNEX TO THE EUROPEAN SEARCH REPORT
## ON EUROPEAN PATENT APPLICATION NO.

EP 21 16 2145

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2021

| Patent document cited in search report | | Publication date | Patent family member(s) | | Publication date |
|---|---|---|---|---|---|
| WO 2017150692 | A1 | 08-09-2017 | CA | 3016326 A1 | 08-09-2017 |
| | | | CN | 108697644 A | 23-10-2018 |
| | | | EP | 3423039 A1 | 09-01-2019 |
| | | | JP | 2017160188 A | 14-09-2017 |
| | | | KR | 20180114170 A | 17-10-2018 |
| | | | US | 2019076361 A1 | 14-03-2019 |
| | | | WO | 2017150692 A1 | 08-09-2017 |
| WO 2020027030 | A1 | 06-02-2020 | NONE | | |
| US 2018085314 | A1 | 29-03-2018 | NONE | | |

EPO FORM P0459

For more details about this annex : see Official Journal of the European Patent Office, No. 12/82

**EP 3 881 831 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5423003 B
- JP 2008292976 A
- JP 4647506 B
- JP 2010102195 A
- JP 2007199463 A